# EUROPEAN PATENT APPLICATION

(11) **EP 4 736 880 A1**
(43) Date of publication of application: **06.05.2026**
(21) Application number: 24831516.0
(22) Date of filing: 24.05.2024
(51) Int. Cl.: A61K 47/34, A61K 9/16, A61K 9/51, A61K 31/713, A61K 31/7088, A61K 47/42, A61K 48/00, A61P 25/28, C12N 15/12, C12N 15/113

(54) **NUCLEIC ACID DELIVERY COMPOSITION AND NUCLEIC-ACID-CONTAINING COMPOSITION**

(30) Priority: 28.06.2023 JP 2023106324
(71) Applicant: Tokushima University, Tokushima-shi, Tokushima 770-8501 (JP)
(72) Inventor: KANAZAWA Takanori, Tokushima-shi, Tokushima 770-8501 (JP); IIOKA Shingo, Shizuoka-shi, Shizuoka 422-8526 (JP); KONDO Hiromu, Shizuoka-shi, Shizuoka 422-8526 (JP)
(74) Representative: Mewburn Ellis LLP
(86) International application number: PCT/JP2024/019180
(87) International publication number: WO 2025/004645

(57) **Abstract**

Provided is a nucleic acid delivery composition which can deliver nucleic acid therapeutics to central nervous system tissues by nasal administration with high efficiency and can form composite particles with good physical stability and uniformity.

The nucleic acid delivery composition of the present invention is a nucleic acid delivery composition used to deliver a nucleic acid to central nervous system tissues or the trigeminal nerve, containing a block copolymer (I) having a polyethylene glycol segment (a) and a polycaprolactone segment (b) and a peptide (II) including 8 to 20 amino acid residues modified with a fatty acid, wherein in the block copolymer (1), the average molecular weight Ma of the polyethylene glycol segment (a) is 1,500 to 2,500 or 3,500 to 6,500, and the average molecular weight Mb of the polycaprolactone segment (b) is 1.5 to 3 times the average molecular weight Ma of the polyethylene glycol segment (a), and the nucleic acid delivery composition is delivered to central nervous system tissues or the trigeminal nerve by nasal administration.

## Description

### TECHNICAL FIELD

The present invention relates to a nucleic acid delivery composition and a nucleic acid-containing composition used to deliver a nucleic acid to a target tissue, and particularly to a nucleic acid delivery composition and a nucleic acid-containing composition which can deliver a nucleic acid to central nervous system tissues such as the brain and spinal cord or the trigeminal nerve by nasal administration.

### BACKGROUND ART

For central nervous system (CNS) diseases such as neurodegenerative diseases, brain tumors, cerebrovascular disorders and brain infectious diseases for which effective therapeutic agents have not existed until now, nucleic acid therapeutics which can knock down CNS disease-related molecules at the mRNA level have been studied. Currently Nusinersen (a medicine for treating spinal muscular atrophy intended for patients with SMN gene mutation) and Tofersen (a medicine for treating amyotrophic lateral sclerosis intended for patients with SOD1 gene mutation), for example, have been developed as nucleic acid therapeutics for CNS diseases. These, however, all are applied to patients by intrathecal administration and have high invasiveness and high burden to patients.

The nose-to-brain route by nasal administration, meanwhile, has attracted attention as a novel drug transport route to the central nervous system. In this route, a drug directly reaches the central nervous system through neural circuits (olfactory nerve and trigeminal nerve) from the nasal mucosa. Therefore, both an efficient drug transport to the central nervous system not passing through the blood-brain barrier and a reduction in adverse effects on peripheral organs, non-target tissues, can be expected. The present inventors have studied a drug delivery technique via the nose-to-brain route and, as one of the study results, found and reported that a compound obtained by binding a predetermined cell-penetrating peptide to a block copolymer in which a polyethylene glycol segment and a hydrophobic polyester segment are connected, on another terminal side of the hydrophobic polyester segment (the terminal side which is not connected to the polyethylene glycol segment) formed nanoparticles having a micelle structure and functioned as a drug delivery composition which delivers a drug to the spinal cord by nasal administration (Patent Literature 1).

### CITATION LIST

### PATENT LITERATURE

Patent Literature 1: Japanese Patent No. 7211603

### SUMMARY OF INVENTION

### TECHNICAL PROBLEM

The nanoparticles formed by the drug delivery composition reported in Patent Literature 1, however, had problems in that the particle size distribution was wide and uniformity was poor and moreover the average particle diameter and zeta potential, for example, were easily changed over days and physical stability was poor.

The present invention therefore has been made in view of the above-described points and an object thereof is to provide a nucleic acid delivery composition which can deliver nucleic acid therapeutics to central nervous system tissues with high efficiency by nasal administration and can form composite particles having good physical stability and uniformity.

The compound used in the drug delivery composition reported in Patent Literature 1 is obtained by binding the block copolymer and the predetermined cell-penetrating peptide and thus steps of, for example, the synthesis reaction thereof and purification have spent time and effort (costs). Therefore, another object of the present invention is to provide a composition which does not require a complex synthesis reaction for the bond between the block copolymer and the peptide and is obtained from components which can be easily acquired.

### SOLUTION TO PROBLEM

The present inventors aimed at highly efficient delivery of nucleic acid therapeutics to central nervous system tissues by nasal administration and diligently studied combinations of block copolymers having various structures and a predetermined functional peptide. Consequently, the present inventors found a nucleic acid delivery composition which can highly efficiently deliver nucleic acid therapeutics to central nervous system tissues and forms composite particles having good physical stability and uniformity. Based on the knowledge, the present invention was completed.

In order to solve the above problems, the nucleic acid delivery composition of the present invention is a nucleic acid delivery composition used to deliver a nucleic acid to central nervous system tissues or the trigeminal nerve, the nucleic acid delivery composition containing a block copolymer (I) having a polyethylene glycol segment (a) and a polycaprolactone segment (b) and a peptide (II) including 8 to 20 amino acid residues modified with a fatty acid, wherein in the block copolymer (I), the average molecular weight Ma of the polyethylene glycol segment (a) is 1,500 to 2,500 or 3,500 to 6,500 and the average molecular weight Mb of the polycaprolactone segment (b) is 1.5 to 3 times the average molecular weight Ma of the polyethylene glycol segment (a) described above, and the nucleic acid delivery composition is delivered to central nervous system tissues or the trigeminal nerve by nasal administration.

The nucleic acid delivery composition of the present invention is formed by a block copolymer (I) having a polyethylene glycol segment (a) and a polycaprolactone segment (b) and a peptide (II) including 8 to 20 amino acid residues modified with a fatty acid. When among these the average molecular weight Ma of the polyethylene glycol segment (a) and the average molecular weight Mb of the polycaprolactone segment (b), forming the block copolymer (I), are within the predetermined ranges, a nucleic acid delivery composition which can deliver nucleic acid therapeutics to central nervous system tissues or the trigeminal nerve with high efficiency by nasal administration is obtained. It should be noted that in the description, the numerical value range of "the lower limit to the upper limit" indicates the numerical value range of "the lower limit or more and the upper limit or less" unless otherwise specified. In addition, the "average molecular weight" in this description indicates the peak top molecular weight (weight average) measured by GPC method based on a polyethylene glycol standard (manufactured by Sigma-Aldrich).

In the nucleic acid delivery composition of the present invention, the average molecular weight Mb of the polycaprolactone segment (b) in the block copolymer (I) described above is preferably 3,500 to 6,500 or 8,000 to 12,000. Because of this, a particularly suitable range is selected as the average molecular weight of the polycaprolactone segment (b).

In the nucleic acid delivery composition of the present invention, the mixing ratio of the block copolymer (I) and the peptide (II) is preferably (I) : (II) = 0.25 : 1 to 2 : 1 by mole ratio. Because of this, a nucleic acid delivery composition which forms composite particles having good physical stability and uniformity is obtained.

In the nucleic acid delivery composition of the present invention, the block copolymer (I) described above is preferably also methoxypolyethylene glycol-poly(ε-caprolactone). Because of this, a particularly suitable block copolymer is selected as the block copolymer (I).

In the nucleic acid delivery composition of the present invention, it is also preferred that the peptide (II) described above be a peptide including cysteine residues, histidine residues and arginine residues and the fatty acid modifying the peptide be stearic acid. Because of this, a particularly suitable peptide is selected as the peptide (II).

In the nucleic acid delivery composition of the present invention, the block copolymer (I) and the peptide (II) preferably form nanoparticles.

The nucleic acid delivery composition of the present invention forms nanoparticles, and the nanoparticles preferably have an average particle diameter of 50 nm or less, a polydispersity index (PDI) of 0.3 or less and a zeta potential of 1 to 20 mV. Because of this, a nucleic acid delivery composition which forms composite particles having good physical stability and uniformity and can highly efficiently deliver a nucleic acid to a target tissue is obtained.

Furthermore, the nucleic acid-containing composition of the present invention is the above-described nucleic acid delivery composition containing a nucleic acid. Because of this, the nucleic acid is stably retained by the nucleic acid delivery composition and thus a nucleic acid-containing composition which can efficiently deliver a nucleic acid to a target tissue is obtained.

In the nucleic acid-containing composition of the present invention, the ratio of the total number of moles of cationic groups (N) derived from the peptide (II) described above and the total number of moles of phosphate groups (P) derived from the nucleic acid (N/P ratio) is preferably 3 to 30. Because of this, a nucleic acid-containing composition which forms composite particles having good physical stability and uniformity and can deliver a nucleic acid to a target tissue with high efficiency is obtained.

The nucleic acid-containing composition of the present invention preferably contains an antisense oligonucleotide, siRNA or mRNA for treating a disease in central nervous system tissues as the nucleic acid. The nucleic acid-containing composition of the present invention is nasally delivered to central nervous system tissues and thus can directly deliver an antisense oligonucleotide, siRNA or mRNA for treating a disease in central nervous system tissues to a target tissue.

The medicine of the present invention has the nucleic acid-containing composition containing the nucleic acid for treating a disease in central nervous system tissues described above as an active ingredient. Because of this, a medicine by which nucleic acid therapeutics for treatment is delivered to central nervous system tissues, target tissues, by nasal administration is obtained. The medicine of the present invention is nasally delivered to central nervous system tissues and thus low invasive treatment can be provided and also adverse effects on non-target tissues can be reduced.

### ADVANTAGEOUS EFFECT OF INVENTION

According to the present invention, it is possible to provide a nucleic acid delivery composition and a nucleic acid-containing composition having good effects as described below:
(1) a nucleic acid such as nucleic acid therapeutics can be delivered to central nervous system (CNS) tissues or the trigeminal nerve, and
(2) nucleic acid therapeutics can be delivered to a target tissue by nasal administration and thus non-invasive treatment can be achieved and adverse effects on non-target tissues can be reduced.

### BRIEF DESCRIPTION OF DRAWING

FIG. 1 is graphs showing the (a) average particle diameter, (b) zeta potential and (c) PDI (polydispersity index) of nanoparticles in nucleic acid delivery compositions prepared in Example 1;
FIG. 2 is graphs showing the (a) average particle diameter, (b) zeta potential and (c) PDI (polydispersity index) of nanoparticles in nucleic acid delivery compositions prepared by changing the mixing mole ratios of the block copolymer (I) and the peptide (II) in Example 1;
FIG. 3 is (a) graphs showing physical properties of nucleic acid delivery compositions prepared using constituents in Test 1-3 and Test 1-5 as the block copolymer (I) over days and (b) graphs showing physical properties of nucleic acid delivery compositions prepared using a constituent in Test 1-5 as the block copolymer (I) over days over a long period of time in Example 1;
FIG. 4 is a graph showing the particle diameter distribution of nanoparticles in nucleic acid (Malat-1-targeting siRNA)-containing compositions prepared in Example 2;
FIG. 5 is graphs showing a target gene knock-down effect in various central nervous system tissues by nasal administration of nucleic acid (Malat-1-targeting siRNA)-containing compositions in Example 3;
FIG. 6 is graphs showing the (a) average particle diameter, (b) zeta potential and (c) PDI (polydispersity index) of nanoparticles in Malat-1-targeting antisense oligonucleotide-containing compositions prepared in Example 4;
FIG. 7 is a graph showing a target gene knock-down effect in various central nervous system tissues by nasal administration of a Malat-1-targeting antisense oligonucleotide-containing composition in Example 5;
FIG. 8 is graphs showing the (a) average particle diameter, (b) zeta potential and (c) PDI (polydispersity index) of nanoparticles in Malat-1-targeting antisense oligonucleotide-containing compositions prepared by a microfluidic device method in Example 6;
FIG. 9 is graphs showing a target gene knock-down effect in various central nervous system tissues by nasal administration of Malat-1-targeting antisense oligonucleotide-containing compositions prepared by a microfluidic device method in Example 7;

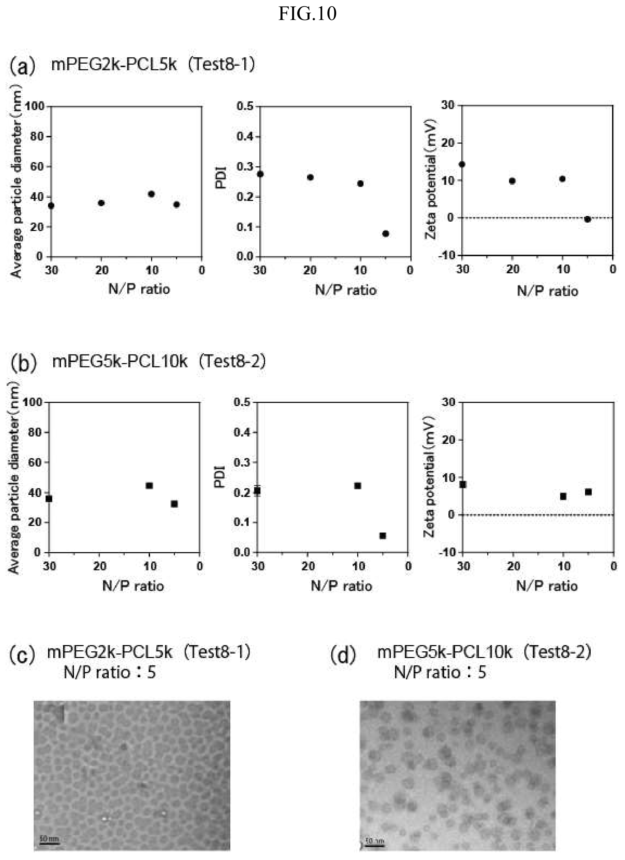

FIG. 10 is (a) graphs showing the average particle diameter, PDI and zeta potential of nanoparticles in hSOD1-targeting antisense oligonucleotide-containing compositions prepared using a constituent in Test 8-1 as the block copolymer (I), (b) graphs showing the average particle diameter, PDI and zeta potential of nanoparticles in hSOD1-targeting antisense oligonucleotide-containing compositions prepared using a constituent in Test 8-2 as the block copolymer (I), (c) an electron microscopic photograph showing nanoparticles in a hSOD1-targeting antisense oligonucleotide-containing composition prepared using a constituent in Test 8-1 as the block copolymer (I), and (d) an electron microscopic photograph showing nanoparticles in a hSOD1-targeting antisense oligonucleotide-containing composition prepared using a constituent in Test 8-2 as the block copolymer (I) in Example 8;
FIG. 11 is graphs showing a target gene knock-down effect in various central nervous system tissues by nasal administration of hSOD1-targeting antisense oligonucleotide-containing compositions to ALS model mice in Example 9;
FIG. 12 is graphs showing the concentration distribution of a hSOD1-targeting antisense oligonucleotide in various central nervous system tissues and the trigeminal nerve by nasal administration of the hSOD1-targeting antisense oligonucleotide-containing composition to Macaca fascicularis in Example 10; and
FIG. 13 is a graph showing a target gene knock-down effect in various central nervous system tissues by nasal administration of a hSOD1-targeting antisense oligonucleotide-containing composition to Macaca fascicularis in Example 10.

### DESCRIPTION OF EMBODIMENT

### [Nucleic acid delivery composition]

The nucleic acid delivery composition of the present invention will now be described in detail. The nucleic acid delivery composition in the present invention is a composition used to deliver a nucleic acid to central nervous system tissues or the trigeminal nerve, and formed by at least a block copolymer (I) having a polyethylene glycol segment (a) and a polycaprolactone segment (b) and a peptide (II) including 8 to 20 amino acid residues modified with a fatty acid. Among these, the average molecular weight Ma of the polyethylene glycol segment (a) in the block copolymer (I) is 1,500 to 2,500 or 3,500 to 6.500 and the average molecular weight Mb of the polycaprolactone segment (b) is 1.5 to 3 times the average molecular weight Ma of the polyethylene glycol segment (a) described above.

The target tissues for the nucleic acid delivery composition of the present invention are central nervous system tissues or the trigeminal nerve. In addition, the nucleic acid delivery composition of the present invention is nasally administered and delivered by the so-called nose-to-brain route. The central nervous system (CNS) tissues are the spinal cord and brain, and examples of the spinal cord include, but not limited to, the cervical spinal cord, thoracic spinal cord, lumbar spinal cord and sacral spinal cord and the like and examples of the brain include, but not limited to, the cerebrum, diencephalon, mesencephalon, pons, cerebellum, medulla oblongata and the like.

### [Block copolymer (I)]

Next, constituents forming the nucleic acid delivery composition of the present invention will be described. In the nucleic acid delivery composition of the present invention, a block copolymer (I) having a polyethylene glycol segment (a) and a polycaprolactone segment (b) is contained.

The polyethylene glycol segment (a) forming the block copolymer (I) is a segment including a polyethylene glycol chain having a repeating structure of the ethyleneoxy group (-CH₂CH₂O-) unit. One terminus of the polyethylene glycol segment (a) is connected to a polycaprolactone segment (b) described below directly or via a binding group. Another terminal side may be the hydroxy group at the terminus of polyethylene glycol or any terminal group obtained by modifying the hydroxy group at the terminus. Specific examples of the terminal group on another side can include, but not limited to, a C1-12 alkoxy group, a C1-12 alkenyloxy group which may have a substituent, a C7-20 aralkyloxy group which may have a substituent and the like. Among these, a C1-6 alkoxy group which may have a substituent is preferred, an unsubstituted C1-3 alkoxy group is more preferred and a methoxy group is particularly preferred.

The polyethylene glycol segment (a) may also have a target-specific molecule via the above-described terminal group. Examples of the target-specific molecule include saccharides, lipid, peptides and proteins and derivatives thereof or folic acid and the like. Among these, a saccharide (e.g. glucose, sucrose, etc.) is mentioned from the viewpoint of efficient delivery to the brain with high specificity. In addition, a ligand for a receptor, an antibody or a peptide or a protein of the fragments thereof is mentioned from the viewpoint of efficient delivery to a target organ with high specificity by interactions with various proteins on nerve cell surfaces of the spinal cord.

The polycaprolactone segment (b) forming the block copolymer (I) is a homopolymer of ε-caprolactone, is a segment including poly(ε-caprolactone) and is a hydrophobic polyester. The above-described polyethylene glycol segment (a) and polycaprolactone segment (b) in the block copolymer (I) may be connected directly or indirectly via a binding group but preferably are directly connected. The binding pattern when the polyethylene glycol segment (a) and the polycaprolactone segment (b) are directly connected is preferably an ester bond formed by the terminal hydroxy group of the polyethylene glycol segment (a) and the terminal carboxy group of the polycaprolactone segment (b). The binding group when the polyethylene glycol segment (a) and the polycaprolactone segment (b) are indirectly connected is not particularly limited as long as it is a group connecting two polymer segments by a chemical bond, and is only needed to be a binding group formed from a functional group which can be bound to the terminal group of the polyethylene glycol segment (a) and the terminal group of the polycaprolactone segment (b). It is preferably a C1-6 alkylene group. The binding pattern of the binding group to the polyethylene glycol segment (a) is preferably an ether bond by the terminal oxygen atom of the poly(oxyethylene) group and the binding pattern thereof to the polycaprolactone segment (b) is preferably an amide bond or an ester bond.

It is important that the block copolymer (I) in the present invention has a structure in which the average molecular weight Mb of the polycaprolactone segment (b) is greater than the average molecular weight Ma of the polyethylene glycol segment (a). Specifically, the average molecular weight Ma of the polyethylene glycol segment (a) is 1,500 to 2,500 or 3,500 to 6,500 and the average molecular weight Mb of the polycaprolactone segment (b) is 1.5 to 3 times the average molecular weight Ma of the polyethylene glycol segment (a) described above and preferably 2 to 2.5 times. Because of this, a nucleic acid delivery composition which can deliver nucleic acid therapeutics to central nervous system tissues or the trigeminal nerve by nasal administration with high efficiency is obtained. More specifically, the average molecular weight Ma of the polyethylene glycol segment (a) is preferably 1,500 to 2,500 or 3,500 to 6,500 and more preferably 1,800 to 2,200 or 4,000 to 6,000. In addition, the average molecular weight Mb of the polycaprolactone segment (b) is adjusted to 1.5 to 3 times the average molecular weight Ma of the polyethylene glycol segment (a) described above and preferably adjusted to 2 to 2.5 times, and thus is preferably 3,500 to 6,500 or 8,000 to 12,000 and more preferably 4,000 to 6,000 or 9,000 to 11,000.

Specific examples of the block copolymer include a polyethylene glycol-poly(ε-caprolactone) copolymer and a monomethoxy polyethylene glycol-poly(ε-caprolactone) copolymer and the like, and a monomethoxy polyethylene glycol-poly(ε-caprolactone) copolymer particularly is preferred. Among these, a monomethoxy polyethylene glycol-poly(ε-caprolactone) copolymer with an average molecular weight of polyethylene glycol of 1,800 to 2,200 and an average molecular weight of poly(ε-caprolactone) of 4,000 to 6,000, and a monomethoxy polyethylene glycol-poly(ε-caprolactone) copolymer with an average molecular weight of polyethylene glycol of 4,000 to 6,000 and an average molecular weight of poly(ε-caprolactone) of 9,000 to 11,000 are particularly preferred.

### [Peptide (II)]

In addition, the nucleic acid delivery composition of the present invention contains a peptide (II) including 8 to 20 amino acid residues modified with a fatty acid. The binding pattern of the peptide (II) is a peptide bond and may be an α-bond, a β-bond or a mixture thereof. The amino acid residues forming the peptide (II) may be a natural amino acid or an unnatural amino acid and both the L-form and D-form thereof can be used without particular limitation. When the peptide (II) is modified with a fatty acid, examples of the fatty acid include C8-24 saturated or unsaturated fatty acids. Among these, the fatty acid is preferably a higher saturated fatty acid (e.g. capric acid, lauric acid, myristic acid, palmitic acid, stearic acid, arachidic acid, behenic acid, etc.) or a higher unsaturated fatty acid (e.g. oleic acid, elaidic acid, linoleic acid, linolenic acid, eleostearic acid, etc.), more preferably a C16-20 saturated fatty acid and particularly preferably stearic acid. The fatty acid can be bound to the amino group at the N terminus or the carboxy group at the C terminus of the peptide directly or via a binding group and more preferably is bound to the amino group at the N terminus of the peptide directly or via a binding group. Examples of the binding group when the peptide is bound to the fatty acid include -CO-, -O-CO-, -NH-CO- and the like and it is particularly preferably -CO-.

The number of amino acid residues in the peptide (II) is 8 to 20, preferably 8 to 15, more preferably 8 to 12 and particularly preferably 9 to 11. The amino acid residues forming the peptide (II) preferably have at least arginine. Arginine is a basic amino acid and positively charged under neutral conditions and thus can form a composite with a nucleic acid having negative charge by electrostatic interactions. Furthermore, arginine has a guanidine unit and thus increases transfection by interactions with a cell membrane or an organelle membrane of, for example, endosome and contributes to improvements in the efficiency of nucleic acid delivery. Because of this, the amino acid residues forming the peptide preferably contain at least arginine and the number of arginine residues to the total number of all residues of the peptide is preferably 30 to 70% and further preferably 40 to 60%. In addition, sulfur amino acids such as cysteine form a disulfide bond between molecules to improve the stability of the nucleic acid delivery composition or the nucleic acid-containing composition, and from this viewpoint, cysteine is also suitably used as an amino acid residue forming the peptide (II). Furthermore, a basic amino acid such as histidine is protonated under acidic biological environments and forms a salt and thus can improve the efficiency of nucleic acid delivery. Therefore, histidine is preferably used as an amino acid residue forming the peptide (II). Because of these, arginine, cysteine and histidine are preferably contained as amino acid residues forming the peptide and the total number of these 3 amino acid residues to the total number of all residues of the peptide is preferably 50 to 100% and further preferably 75 to 100%.

The peptide (II) is particularly preferably STR (stearic acid)-CHHRRRRHHC (SEQ ID NO:1) from the N terminus. In addition, the cell-penetrating peptide (CPP) may be a peptide obtained by modifying a known peptide with a fatty acid. Other examples of the cell-penetrating peptide include TAT peptide, oligoarginine such as octaarginine, (PRP)ₙ, penetratin and the like.

The mixing proportion of the block copolymer (I) and the peptide (II), the above-described constituents, in the nucleic acid delivery composition of the present invention is preferably (I) : (II) = 0.25 : 1 to 2 : 1, more preferably (I) : (II) = 0.4 : 1 to 1.5 : 1 and particularly preferably (I) : (II) = 0.5 : 1 to 1.25 : 1 by mole ratio. A nucleic acid delivery composition forming nanoparticles which show suitable physical properties (average particle diameter and zeta potential) and have good physical stability and uniformity is obtained by the above ratio.

In the nucleic acid delivery composition of the present invention, the block copolymer (I) and the peptide (II) form a composite to form nanoparticles. It is considered that the polycaprolactone segment (b) of the block copolymer (I) and a fatty acid group modifying the peptide (II) are associated by hydrophobic interactions to form a micelle structure (micelle particles) and thus nanoparticles are formed; however, the nanoparticles are not limited to the micelle particles and may be nanoparticles having other structures. The average particle diameter of nanoparticles formed by the nucleic acid delivery composition of the present invention is preferably 50 nm or less, more preferably 40 nm or less and particularly preferably 30 nm or less. When the average particle diameter of nanoparticles is within this range, the properties of nucleic acid delivery to central nervous system tissues or the trigeminal nerve, a target tissue, and distribution efficiency in the target tissue can be increased. It should be noted that the average particle diameter in this description is an average particle diameter by a dynamic light scattering method and can be measured using a light scattering particle diameter measurement device (e.g. Zetasizer Ultra manufactured by Malvern Panalytical Ltd.) The light scattering particle diameter measurement device can measure a cumulant average particle diameter and a mass average particle diameter and the cumulant average particle diameter measured by Zetasizer Ultra manufactured by Malvern Panalytical Ltd. is preferably used.

Furthermore, the polydispersity index (PDI) measured by the dynamic light scattering method and found in the analysis of a cumulant method is an index to evaluate the width (spread) of particle diameter distribution. The polydispersity index of nanoparticles formed by the nucleic acid delivery composition of the present invention is preferably 0.3 or less, more preferably 0.2 or less and particularly preferably 0.15 or less. When the PDI of nanoparticles is within this range, a nucleic acid delivery composition forming nanoparticles having good physical stability and uniformity is obtained.

In addition, the zeta potential of nanoparticles formed by the nucleic acid delivery composition of the present invention is preferably 1 to 30 mV, more preferably 1 to 20 mV and particularly preferably 5 to 15 mV. When the zeta potential of nanoparticles is within this range, the properties of nucleic acid delivery to central nervous system tissues or the trigeminal nerve, a target tissue, and distribution efficiency in the target tissue can be increased.

It should be noted that components other than the above-described constituents (I) and (II) can be mixed in the nucleic acid delivery composition of the present invention as needed. Specifically, for example, pharmaceutically acceptable additives commonly used may be contained to prepare a nucleic acid-containing composition in a nasal dosage form. An excipient, an expander, a filler, a binder, a wetting agent, a lubricant, a glidant, a surfactant, a disintegrator, a solvent, a solubilizer, a dispersant, a buffer, a stabilizer, a suspending agent, a solubilizing aid, a preservative, an antiseptic, a flavoring agent, a pain reliever, an isotonic agent, a thickener, a pigment, a fragrance and the like, for example, can be used as additives. The additives may be used individually or two or more additives may be used in combination in any ratio. The details such as the type and amount of these other components used can be appropriately decided by those skilled in the art depending on the purpose, uses and usage of the nucleic acid delivery composition or the nucleic acid-containing composition of the present invention.

Next, the method for producing the nucleic acid delivery composition of the present invention will be described. A block copolymer solution obtained by dissolving or dispersing the block copolymer (I) in a water-soluble organic solvent and a peptide solution obtained by dissolving or dispersing the peptide (II) in an aqueous solution are each prepared and the nucleic acid delivery composition of the present invention can be obtained by mixing the block copolymer solution and the peptide solution and stirring the obtained mixture and then removing the organic solvent, but the method is not limited to the above. It should be noted that alcohols such as ethanol and methanol, ethers such as tetrahydrofuran and 1,4-dioxane, acetone, acetonitrile and the like can be used as the organic solvent used to prepare the block copolymer solution. Water itself and moreover buffer solutions (PBS, HEPES, etc.), saline, an aqueous solution of glucose and the like can be used as the aqueous solution used to prepare the peptide solution. In order to mix the block copolymer solution and the peptide solution, all known mixing methods can be used as the method for producing the nucleic acid delivery composition and the nucleic acid-containing composition. Examples of the method for removing the organic solvent from the mixed solution include a method using an ultrafiltration membrane (e.g. a method using dialysis or a centrifugal ultrafiltration device, etc.) and a solvent distillation method and the like and the method using an ultrafiltration membrane is preferably used. In addition, the pH of the respective component solutions and a mixed solution thereof can be appropriately adjusted without inhibiting the ability of forming nanoparticles. The pH is preferably 5 to 9, more preferably 6.5 to 8.0 and further preferably 7.0 to 8.0. The pH can be easily adjusted by using a buffer solution as a solvent. The concentration of the respective component solutions and a salt of the buffer solution in the mixed solution thereof can be appropriately adjusted without inhibiting the ability of forming particles.

The nucleic acid delivery composition of the present invention can be further prepared by a microchannel device method in which a plurality of solutions are mixed in a mixing channel having a special channel structure. A dispersion liquid containing the nucleic acid delivery composition of the present invention can be easily obtained particularly using an iLiNP microchannel chip made of quartz glass (manufactured by Lilac pharma). Specifically, a dispersion liquid containing nanoparticles of the nucleic acid delivery composition can be produced by mixing the block copolymer solution and the peptide solution in the iLiNP microchannel chip. The average particle diameter of nanoparticles of the nucleic acid delivery composition obtained can be controlled by changing the polymer concentration in the block copolymer solution, the peptide concentration in the peptide solution, the total flow rate (TFR) and flow rate ratio (FRR) of the block copolymer solution and the peptide solution. The nanoparticles of the nucleic acid delivery composition and solvents used for the respective solutions are contained in the dispersion liquid produced by the method; however, the respective solvents are can be removed and replaced by a treatment such as dialysis or ultrafiltration to concentrate the dispersion liquid.

### [Nucleic acid-containing composition]

The nucleic acid-containing composition according to the present invention is a composition in which a nucleic acid is contained in the above-described nucleic acid delivery composition. The nucleic acid-containing composition can deliver a nucleic acid molecule to central nervous system tissues or the trigeminal nerve via the nose-to-brain route by nasal administration and introduce it into cells. Using the present invention, a nucleic acid having a physiological activity can be delivered to the trigeminal nerve and central nervous system tissues while avoiding various nucleases in living bodies, the nucleic acid is introduced into cells and allowed to escape from endosome and thus the nucleic acid can be released into cells and the function of the nucleic acid can be displayed.

In the present invention, the nucleic acid contained in the nucleic acid-containing composition is not particularly restricted and examples thereof include DNA, RNA, other natural nucleic acids and modified nucleic acids thereof and the like. In addition, the nucleic acid may be a single-stranded nucleic acid or a double-stranded nucleic acid. Examples of the nucleic acid include antisense nucleic acids, siRNAs, miRNAs, mRNAs, shRNAs, pre-miRNAs, pri-miRNAs, plasmid DNAs, decoy nucleic acids, ribozymes, DNA aptamers, RNA aptamers, DNA enzymes and various suppressor genes and the like. Among these, a nucleic acid for treating central nervous system tissues is suitably used in the present invention, and RNA (siRNA or miRNA) or an antisense nucleic acid having an action of suppressing the expression of a target gene using RNA interference particularly is preferably used, and siRNA or an antisense nucleic acid is more preferably used.

The ratio of the peptide (II) and the nucleic acid contained in the nucleic acid-containing composition of the present invention is represented by the N/P ratio provided by the ratio of the total number of moles (N) of cationic groups derived from the peptide (II) and the total number of moles (P) of phosphate groups (anionic group) derived from the nucleic acid (a value obtained by dividing the N value by the P value). In the nucleic acid-containing composition of the present invention, the N/P ratio is preferably 1 to 50, more preferably 3 to 30 and further preferably 5 to 20. When the N/P ratio is within the above-described range, both improvements in physical stability and improvements in transfection rate can be achieved.

In the present invention, when the nucleic acid-containing composition is prepared by adding a nucleic acid to the nucleic acid delivery composition containing the block copolymer (I) and the peptide (II), a composite is preferably formed by interactions between these constituents. Because the peptide (II) has a modifying fatty acid group, a nucleic acid, an anionically charged body, and the peptide (II), a cationically charged body, are associated by electrostatic interactions and moreover the polycaprolactone segment (b) in the block copolymer (I) and the fatty acid group of the peptide (II) are associated by hydrophobic interactions, and thus nanoparticles having a micelle structure are preferably formed. The physical properties of the nanoparticles formed by the nucleic acid-containing composition are the same as of the above-described nucleic acid delivery composition and the average particle diameter of the nanoparticles is preferably 50 nm or less, more preferably 40 nm or less and particularly preferably 30 nm or less. In addition, the polydispersity index (PDI) is preferably 0.3 or less, more preferably 0.2 or less and particularly preferably 0.15 or less. Then, the zeta potential of the nanoparticles is preferably 1 to 30 mV, more preferably 1 to 20 mV and particularly preferably 5 to 15 mV.

As the method for preparing the nucleic acid-containing composition according to the present invention, for example, the composition can be prepared by dissolving or dispersing each of the block copolymer (I), the peptide (II) and the nucleic acid and mixing the obtained solutions. From the viewpoint that a nucleic acid-containing composition forming nanoparticles with a particle diameter of about 50 nm or less particularly can be produced with high reproducibility, a dispersion liquid in which a nucleic acid-containing composition is dispersed in water can be obtained by preparing the nucleic acid delivery composition dispersed in water by the above-described method and then mixing an aqueous solution of the nucleic acid in the composition. Furthermore, the obtained nucleic acid-containing composition solution can be appropriately used for operations such as dilution, stirring, ultrasonic irradiation, dialysis and concentration.

In addition to the above-described components, another drug other than the nucleic acid, a pharmacologically acceptable carrier for general preparation, and additives acceptable as additives for pharmaceutical products can be also contained in the nucleic acid-containing composition of the present invention.

The medicine according to the present invention has the nucleic acid-containing composition containing the nucleic acid for treating central nervous system tissues described above as an active ingredient. Because of this, the medicine can be used for the prevention, improvement or treatment of pathologic conditions, symptoms or diseases related to, for example, damages and malfunction of central nervous system tissues. In the treatment and the improvements in the pathologic conditions the prevention and treatment for prognosis of the diseases and pathologic conditions in central nervous system tissues are also contained. The diseases in central nervous system tissues include, for example, amyotrophic lateral sclerosis (ALS), spinocerebellar degeneration, spinal muscular atrophy, primary lateral sclerosis, spinal and bulbar muscular atrophy, chronic pain, spinal cord injury, spinal tumors, brain tumors, cerebrovascular disorders, Parkinson's disease, virus infections, Alzheimer's disease and dementia and the like or symptoms or diseases related thereto. The medicine containing the nucleic acid-containing composition of the present invention as an active ingredient is used for, for example, the treatment to and improvement in one or a plurality of these pathologic conditions, symptoms or diseases and the suppression and prevention of the symptoms and pathologic conditions.

The administration route for the medicine containing the nucleic acid-containing composition according to the present invention is nasal administration. The nucleic acid therapeutics contained in the nucleic acid delivery composition can be efficiently delivered to central nervous system tissues or the trigeminal nerve by nasal administration. Therefore, the nucleic acid-containing composition and medicine according to the present invention can be in any dosage form suitable for nasal administration. Examples of the dosage form suitable for nasal administration include liquid preparation, aerosol preparation, powder preparation and the like. The dosage or effective intake of the medicine containing the nucleic acid-containing composition according to the present invention as an active ingredient varies depending on the nucleic acid contained in the nucleic acid delivery composition and the amount thereof contained, intended treatment effects, administration method, administration subjects and dosage forms and thus is not particularly limited.

### EXAMPLES

The present invention will now be described in more detail by way of Examples and Comparative Examples thereof. It should be noted, however, that the present invention is not limited to these Examples. The constituents of nucleic acid delivery compositions and nucleic acid-containing compositions prepared in Examples and Comparative Examples below are shown below.

### Block copolymer (I):

- Methoxypolyethylene glycol-poly(ε-caprolactone), {molecular weight of polyethylene glycol segment by HNMR: 1,800 to 2,200, molecular weight of polycaprolactone segment by HNMR: 4,000 to 6,000 (hereinafter referred to as "mPEG2k-PCL5k")} manufactured by Sigma-Aldrich (product number 900648, Methoxy poly(ethylene glycol)-block-poly(ε-caprolactone), 2k-5k);
- Methoxypolyethylene glycol-poly(ε-caprolactone), {molecular weight of polyethylene glycol segment by HNMR: 4,000 to 6,000, molecular weight of polycaprolactone segment by HNMR: 9,000 to 11,000 (hereinafter referred to as "mPEG5k-PCL10k")} manufactured by Sigma-Aldrich (product number 900672, Methoxy poly(ethylene glycol)-block-poly(ε-caprolactone), 5k-10k); and
- Methoxypolyethylene glycol-poly(ε-caprolactone), {molecular weight of polyethylene glycol segment by HNMR: 1,800 to 2,200, molecular weight of polycaprolactone segment by HNMR: 1,800 to 2,200 (hereinafter, referred to as "mPEG2k-PCL2k")} manufactured by Sigma-Aldrich (product number 900649, Methoxy poly(ethylene glycol)-block-poly(ε-caprolactone), 2k-2k).

### Peptide (II):

- Peptide having the N terminal amino group modified with stearic acid including the amino acid sequence: CHHRRRRHHC (SEQ ID NO:1) (hereinafter referred to as "STR-CH₂R₄H₂C") manufactured by BEX CO., LTD.; and
- Peptide not modified with stearic acid including the amino acid sequence: CHHRRRRHHC (SEQ ID NO:2) (hereinafter referred to as "HO-CH₂R₄H₂C") manufactured by BEX CO., LTD.

### [Example 1]

### 1. Preparation of nucleic acid delivery composition

In this Example, nucleic acid delivery compositions were prepared by a method including mixing and stirring-ultrafiltration. The block copolymer (I) and the peptide (II) forming the nucleic acid delivery compositions were combinations in Test 1-1 to 1-6 shown in Table 1 below and a plurality of nucleic acid delivery compositions were prepared by changing the mixing mole ratio of the block copolymer (I) and the peptide (II).

**[Table 1]**

| Test No. | Block copolymer (I) | Peptide (II) |
|---|---|---|
| Test 1-1 | mPEG2k-PCL2k | STR-CH₂R₄H₂C |
| Test 1-2 | mPEG2k-PCL2k | HO-CH₂R₄H₂C |
| Test 1-3 | mPEG2k-PCL5k | STR-CH₂R₄H₂C |
| Test 1-4 | mPEG2k-PCL5k | HO-CH₂R₄H₂C |
| Test 1-5 | mPEG5k-PCL10k | STR-CH₂R₄H₂C |
| Test 1-6 | mPEG5k-PCL10k | HO-CH₂R₄H₂C |

The preparation method is as follows. First, the block copolymer (I) was dissolved in tetrahydrofuran (THF) to prepare a block copolymer solution. Meanwhile, the peptide (II) was dissolved in an HEPES buffer solution (10 mM, pH 7.4) to prepare a peptide solution. In addition, (±)dithiothreitol (DTT) was dissolved in an HEPES buffer solution (10 mM, pH 7.4) to prepare a 100 mM DTT/HEPES solution.

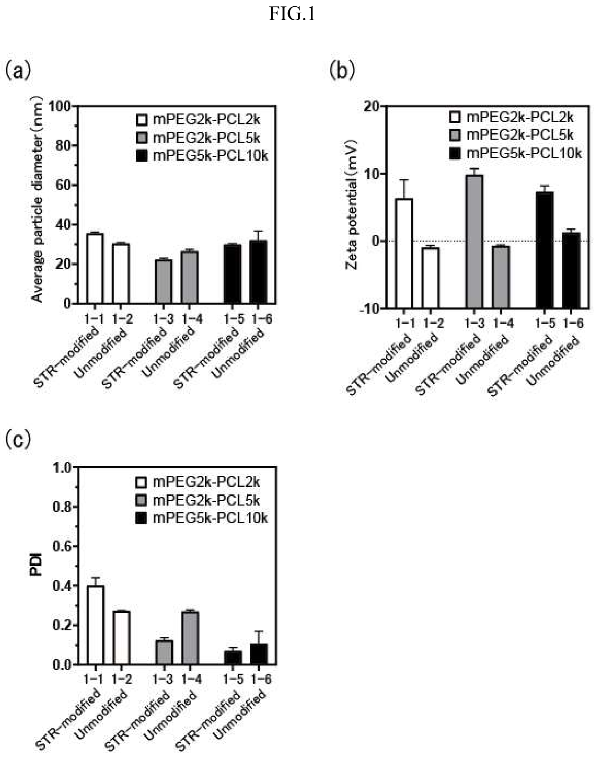

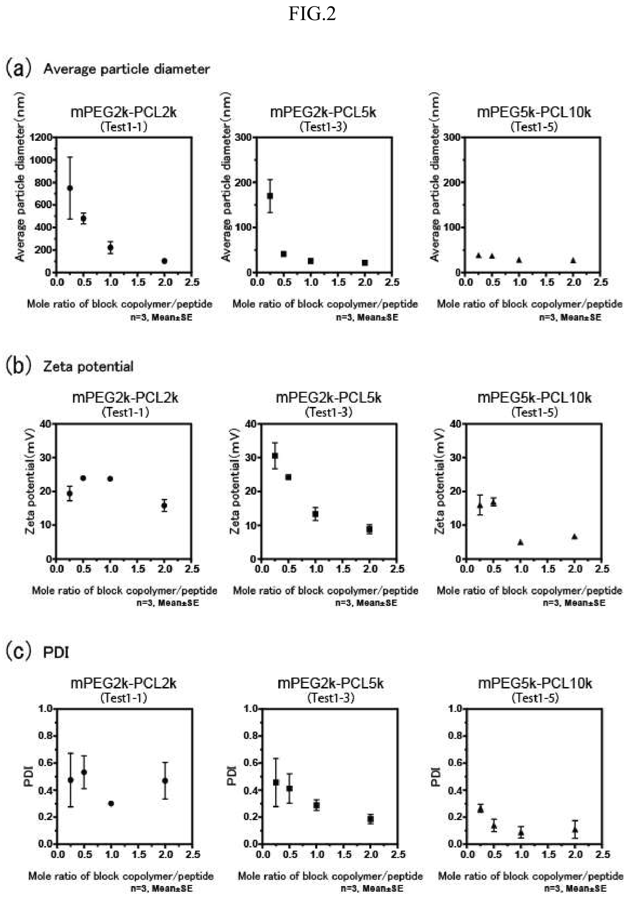

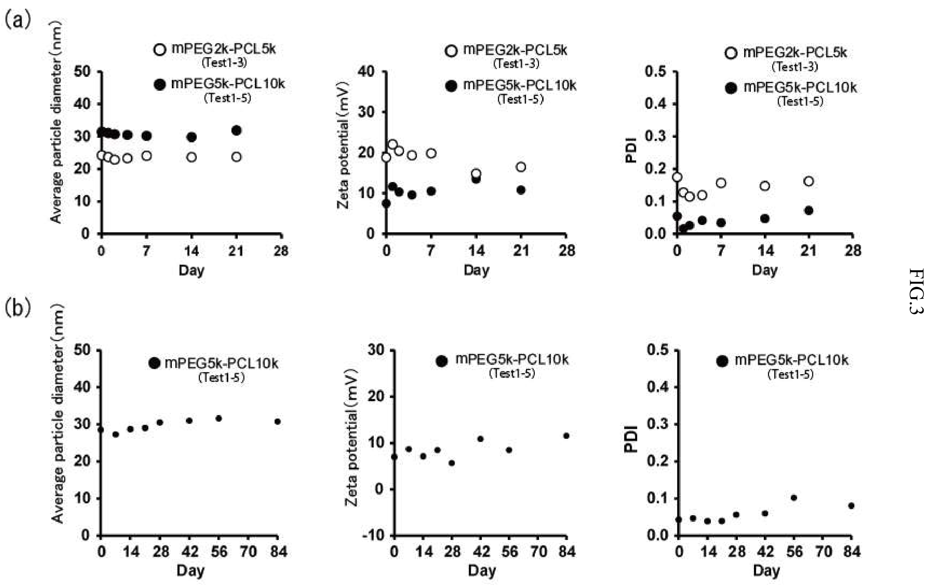

Using the respective solutions prepared as described above, composite particles of the block copolymer (I) and the peptide (II) were prepared. The preparation was carried out under conditions of 20°C. First, the DTT/HEPES solution was added to the peptide solution so that DTT was 61.7 nmol with respect to 1 nmol peptide (II) and the HEPES buffer solution (10 mM, pH 7.4) was added until the total amount of the solution was 150 µL. This peptide solution was vortexed for stirring and mixing and then left to stand for 15 minutes. After standing, 60 µL of the block copolymer solutions having various prepared concentrations were each added dropwise thereto so that the mixing mole ratio of the block copolymer (I) was in a range of 0.25 mol to 2 mol with respect to 1 mol peptide (II), and the obtained mixture was vortexed for mixing and stirring and then left to stand for 15 minutes. Next, 290 µL of the HEPES buffer solution (10 mM, pH 7.4) was put in a pre-treated centrifugal filter unit (Amicon Ultra, membrane NMWL 3,000-0.5 mL, manufactured by Merck KGaA) and the mixed liquid of the block copolymer solution and the peptide solution was added dropwise thereto. After that, the obtained mixture was centrifuged under conditions of 20°C and 4,700 G using a high speed refrigerated micro centrifuge (model number: MDX-310 manufactured by TOMY SEIKO CO., LTD.) and ultrafiltration treatment was carried out. Dilution by the HEPES buffer solution (10 mM, pH 7.4) until the concentration of THF remaining in the solution was 0.1% or less and centrifugation were carried out and the solution in the filter unit was concentrated to 100 µL. The whole amount of the solution in the filter unit was recovered to obtain a nucleic acid delivery composition having composite particles of the block copolymer (I) and the peptide (II) dispersed in the solution. The physical properties such as the average particle diameter (the cumulant average particle diameter by the dynamic light scattering method), polydispersity index (PDI) and zeta potential (particle charge) of particles in the nucleic acid delivery composition were measured by a light scattering analyzer (Zetasizer Ultra manufactured by Malvern Panalytical Ltd.) The measurement temperature was 25°C. The results are shown in FIG. 1 and 2. In addition, the prepared nucleic acid delivery composition was transferred to a glass vial and stored under refrigeration at 4°C and the physical properties of composite particles in the nucleic acid delivery composition were measured over days to evaluate storage stability of the nucleic acid delivery composition. The results are shown in FIG. 3.

FIG. 1 shows the physical properties of nucleic acid delivery compositions obtained by the combinations of the block copolymer (I) and the peptide (II) in Test 1-1 to 1-6. It should be noted that the mole ratios of the block copolymer (I) and the peptide (II) in the nucleic acid delivery compositions shown in the graphs were block copolymer (I) : peptide (II) = 1 : 1 for mPEG2k-PCL2k and mPEG2k-PCL5k and block copolymer (I) : peptide (II) = 0.5 : 1 for mPEG5K-PCL10K. In addition, the n number was 3.

As shown in FIG. 1(a), nanoparticles with an average particle diameter of less than 40 nm were formed in the nucleic acid delivery compositions in every test plot. About the zeta potential, meanwhile, in the nucleic acid delivery compositions prepared using a basic peptide modified with stearic acid, a positively charged zeta potential derived from the basic peptide was shown while in the compositions prepared using a peptide not modified with stearic acid, a zeta potential around neutral charge was shown in every molecular weight polymer as shown in FIG. 1(b). This suggested that nanoparticles of a composite formed from a polymer and a peptide were formed using a peptide modified with stearic acid while when using unmodified peptide nanoparticles were formed only by a polymer. This revealed that modification of a peptide with a fatty acid was important to form nanoparticles by a composite of a polymer and a peptide. FIG. 1(c) showed that the value of the polydispersity index (PDI) was reduced in the order of mPEG2k-PCL2k > mPEG2k-PCL5k > mPEG5K-PCL10K and nanoparticles with a uniform particle diameter were formed.

FIG. 2 shows the physical properties of nucleic acid delivery compositions prepared by changing the mixing mole ratio of the block copolymer (I) and the peptide (II) in the combination of the block copolymer (I) and the stearic acid-modified peptide (II) in Test 1-1, 1-3 and 1-5. As shown in FIG. 2(a), the average particle diameters of composite particles of nucleic acid delivery compositions varied depending on the type and the mixing mole ratio of the block copolymer (I). Specifically, composite particles of the nucleic acid delivery compositions formed by mPEG2k-PCL2k (Test 1-1) had a larger average particle diameter than of other particles and had an average particle diameter of about 100 nm even when the mole ratio of block copolymer (I)/peptide (II) was 2.0. In composite particles of the nucleic acid delivery compositions formed by mPEG2k-PCL5k (Test 1-3), meanwhile, the average particle diameter was stably 50 nm or less when the mole ratio of block copolymer (I)/peptide (II) was 0.5 or more. In composite particles of the nucleic acid delivery compositions formed by mPEG5k-PCL10k (Test 1-5), furthermore, the average particle diameter was stably 50 nm or less in every mole ratio of block copolymer (I)/peptide (II).

About the zeta potential of composite particles of the nucleic acid delivery compositions, meanwhile, the charge tended to decrease by increases in the mole ratio of block copolymer (I)/peptide (II) in particles formed using every molecular weight polymer as shown in FIG. 2(b). The absolute values of charges were mPEG2k-PCL2k > mPEG2k-PCL5k > mPEG5k-PCL10k and the zeta potential largely decreased with increases in the mole ratio of block copolymer (I)/peptide (II) particularly in composite particles of the nucleic acid delivery compositions formed by mPEG2k-PCL5k and mPEG5k-PCL10k. About the polydispersity index (PDI) of composite particles of the nucleic acid delivery compositions, the PDI as an index for the uniformity of particle diameter was greater, 0.3 or more, in particles obtained using mPEG2k-PCL2k as shown in FIG. 2(c) and production reproducibility was also bad. In particles of the nucleic acid delivery compositions formed using mPEG2k-PCL5k, meanwhile, the PDI decreased by increases in the mole ratio of block copolymer (I)/peptide (II) and showed 0.2 or less as the minimum. In particles of the nucleic acid delivery compositions formed using mPEG5k-PCL10k, particularly, the PDI showed very high uniformity, about 0.1.

FIG. 3 shows the physical properties over days when the nanoparticles of the nucleic acid delivery compositions prepared by the combinations of the block copolymer (I) and the peptide (II) in Test 1-3 and 1-5 were stored for a long period of time. It should be noted that the mole ratios of the block copolymer (I) and the peptide (II) in the nucleic acid delivery compositions shown in the graphs were block copolymer (I) : peptide (II) = 1 : 1 for mPEG2k-PCL5k (Test 1-3) and block copolymer (I) : peptide (II) = 0.5 : 1 for mPEG5k-PCL10k (Test 1-5).

According to the graphs in FIG. 3(a), composite particles of nucleic acid delivery compositions formed using mPEG2k-PCL5k and mPEG5k-PCL10k as a constituent maintained a good average particle diameter, zeta potential and PDI over 3 weeks and showed high physical stability. According to FIG. 3(b), nanoparticles of the nucleic acid delivery composition formed using mPEG5k-PCL10k as a constituent showed a stable average particle diameter, zeta potential and PDI over about 3 months. The results revealed that the nucleic acid delivery compositions formed using mPEG5k-PCL10k and mPEG2k-PCL5k as a constituent had high physical stability and good uniformity. This suggested that the nucleic acid delivery composition not only could be distributed as a medicine but also is an element important for administration to living bodies and the composition could stably deliver a nucleic acid to central nervous system tissues when it was nasally administered.

### [Example 2]

### 2. Preparation of nucleic acid (siRNA)-containing composition

In this Example, two nucleic acid-containing compositions containing siRNA as a nucleic acid were prepared. The block copolymer (I), peptide (II) and nucleic acid forming the nucleic acid-containing compositions are shown in Tables 2 and 3 below. In this Example, siRNA (BIONEER CORPORATION) designed to target Malat-1 (metastasis associated lung adenocarcinoma transcript 1) gene expressed constitutively in the brain was used as the nucleic acid. In addition, the ratio of nucleic acid contained, "N/P ratio," in the nucleic acid-containing compositions was calculated by the following formula:
- N/P ratio = the number of cationic groups in the nucleic acid delivery composition/the number of anionic groups in the nucleic acid = (the number of N⁺ x the mole number of peptide in the peptide (II) forming the nucleic acid delivery composition)/(PO₄⁻ in the nucleic acid x the mole number of nucleic acid x the number of strands).

**[Table 2]**

| Test No. | Block copolymer (I) (mole ratio) | Peptide (II) (mole ratio) | Nucleic acid | N/P ratio |
|---|---|---|---|---|
| Test 2-1 | mPEG2k-PCL2k (1 mol) | STR-CH₂R₄H₂C (1 mol) | Malat- 1-targeting siRNA | 30 |
| Test 2-2 | mPEG2k-PCL5k (1 mol) | STR-CH₂R₄H₂C (1 mol) | Malat-1-targeting siRNA | 30 |

**[Table 3]**

| Malat-1-targeting siRNA | | |
|---|---|---|
| Sense chain | 5'-AUCCGCGCGAUAGUACGUATT-3' | SEQ ID NO:3 |
| Antisense chain | 5'-UACGUACUAUCGCGCGGAUTT-3' | SEQ ID NO:4 |

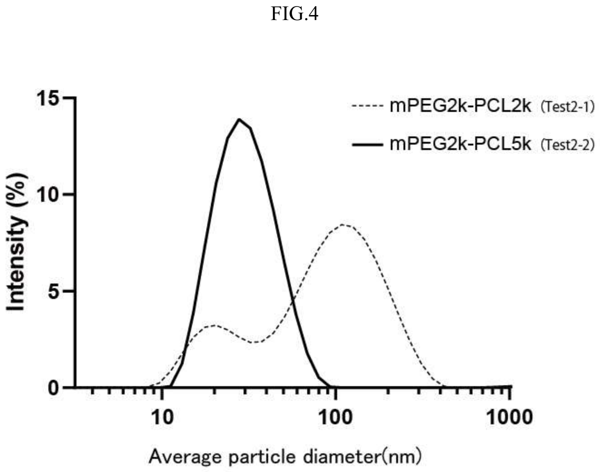

As the nucleic acid (siRNA), a 1 mM nucleic acid solution was prepared using nuclease-free water and stored at -80°C. A nucleic acid delivery composition was prepared from the block copolymer (I) and the peptide (II) by the same procedure and method as in Example 1 to obtain a dispersion liquid in which composite particles of the nucleic acid delivery composition was dispersed in the solution. The HEPES buffer solution (10 mM, pH 7.4) was added to the nucleic acid solution so that the amount was equal to the amount of the dispersion liquid. This nucleic acid solution was added dropwise in an equal amount to the dispersion liquid in which composite particles of the nucleic acid delivery composition were dispersed and the obtained mixture was then mildly vortexed and left to stand for 30 minutes to obtain a nucleic acid-containing composition in which composite particles of the nucleic acid, the block copolymer (I) and the peptide (II) were dispersed in the solution. The physical properties such as the average particle diameter (the cumulant average particle diameter by the dynamic light scattering method), polydispersity index (PDI) and zeta potential (particle charge) of composite particles in the obtained nucleic acid-containing compositions were measured by a light scattering analyzer (Zetasizer Ultra manufactured by Malvern Panalytical Ltd.) The measurement temperature was 25°C. In addition, the form of composite particles in the nucleic acid-containing compositions was observed by a transmission electron microscope (TEM). The results are shown in Table 4 and FIG. 4.

**[Table 4]**

| Test No. | Block copolymer (I) | Average particle diameter (nm) | Zeta potential (mV) | PDI |
|---|---|---|---|---|
| Test 2-1 | mPEG2k-PCL2k | 59.9 | 16.73 | 0.482 |
| Test 2-2 | mPEG2k-PCL5k | 29.0 | 15.03 | 0.190 |

According to the results in FIG. 4 and Table 4, in the nucleic acid-containing composition prepared using mPEG2k-PCL2k as the block copolymer (I), not only the average particle diameter was greater, 60 nm, but also the particle diameter distribution was broader and two peaks were shown as shown in the graph in FIG. 4 (Test 2-1). Therefore, the PDI was also a greater value, 0.482. In the nucleic acid-containing composition prepared using mPEG2k-PCL5k as the block copolymer (I) (Test 2-2), meanwhile, the average particle diameter was smaller, 30 nm, and the width of the particle diameter distribution was also narrower and a single peak was shown. Therefore, the PDI showed a smaller value, 0.190. This revealed that the nucleic acid-containing composition prepared using mPEG2k-PCL5k showed very good physical properties as with the case of the nucleic acid delivery composition before adding the nucleic acid.

### [Example 3]

### 3. Study of knock-down effect of nucleic acid (siRNA)-containing composition by nasal administration

In this Example, the nucleic acid (Malat-1-targeting siRNA)-containing compositions in Test 2-1 and Test 2-2 prepared in Example 2 described above were nasally administered to mice and the effect of nucleic acid delivery was examined by verifying whether or not Malat-1 gene constitutively expressed in the brain was knocked down by siRNA using the expression level of Malat-1 gene.

An animal test was performed in accordance with a protocol approved based on the animal testing regulations of University of Shizuoka. For the administration of the siRNA-containing compositions, 5- to 6-week-old ICR mice (manufactured by Japan SLC, inc.) were used. The ICR mice were anesthetized by inhalation with isoflurane using a laboratory animal anesthesia system SN-487. The anesthetic concentration was 4% for induction and 2% for maintenance. The mice were put on their backs and a Malat-1-targeting siRNA-containing composition prepared as Test 2-1 or Test 2-2 was nasally administered to the mice using an anesthesia mask for nasal administration (model number: SN-487-70-09 manufactured by SHINANO manufacturing Co., Ltd.) under inhalation anesthesia. The nasal administration was carried out by administering each 2 µL, 20 µL in total, to right and left nasal cavities alternately at an interval of one minute and was single administration. The dosage of the Malat-1-targeting siRNA was 2 nmol (26.5 µg)/20 µL. The composition concentrations and N/P ratio of the administered Malat-1-targeting siRNA-containing compositions are each shown below:
- Malat-1-targeting siRNA: 0.1 nmol/µL,
- Concentration of block copolymer (I) (mPEG2k-PCL2k or mPEG2k-PCL5k): 31.5 nmol/µL,
- Concentration of peptide (II) (STR-CH₂R₄H₂C): 31.5 nmol/µL, and
- N/P ratio: 30.

The mice were euthanized at an anesthetic concentration of 5% 48 hours after completion of nasal administration and then the brain and spinal cord and peripheral tissues were extracted. The extracted tissues were each washed using 0.9% saline. The brain was divided into the olfactory bulb, cerebral cortex, hippocampus, cerebellum and brain stem (combined part of the mesencephalon, pons and medulla oblongata). To the extracted and divided sites, an RNA extraction reagent (RNAiso manufactured by Takara Bio Inc.) was added, followed by homogenization to extract crude total RNA. To the crude total RNA, DNase (product number: 313-03161 manufactured by NIPPON GENE CO., LTD.) was added and purification was carried out by DNase treatment. To the purified total RNA, a reverse transcriptase (ReverTra Ace (registered trademark) manufactured by TOYOBO Co., Ltd.) was added and a reverse transcription reaction was carried out using a thermal cycler under conditions at 42°C for 40 seconds -> at 99°C for 5 seconds to prepare cDNA.

The obtained cDNA was subjected to qPCR by SYBR (registered trademark) Green assay. To 2 µL of the prepared cDNA, a qPCR reaction solution (SYBR green manufactured by Promega K.K.), the mMALAT-1 primer set of SEQ ID NO:5 and 6 shown in Table 4 below and nuclease-free water were added and the obtained mixture was mixed by pipetting and subjected to qPCR using a real-time PCR analysis system (CFX connect manufactured by Bio-Rad Laboratories, Inc.) after spin down. The qPCR was carried out by increasing temperature at 95°C for 30 minutes for polymerase activation and then repeating [95°C, 30 sec -> 58°C, 10 sec -> 72°C, 20 sec] for 40 cycles. Meanwhile, human glyceraldehyde-3-phosphate dehydrogenase (GAPDH), a housekeeping gene, was selected as an internal reference and qPCR was carried out using the primer set of SEQ ID NO:7 and 8 shown in Table 5 below in place of the mMALAT-1 primer set described above.

**[Table 5]**

| Primer sequence | | |
|---|---|---|
| mMALAT-1 Forward | 5'-GTTACCAGCCCAAACCTCAA-3' | SEQ ID NO:5 |
| mMALAT-1 Reverse | 5'-CGATGTGGCAGAGAAATCAC-3' | SEQ ID NO:6 |
| GAPDH Forward | 5'-TCAACAGCAACTCCCACTCTTCCA-3' | SEQ ID NO:7 |
| GAPDH Reverse | 5'-ACCCTGTTGCTGTAGCCGTATTCA-3' | SEQ ID NO:8 |

The ΔCt value was calculated using the Ct value obtained from the results of qPCR by the following formula:
- ΔCt value = Ct value of target gene (Malat-1) - Ct value of reference gene (GAPDH).

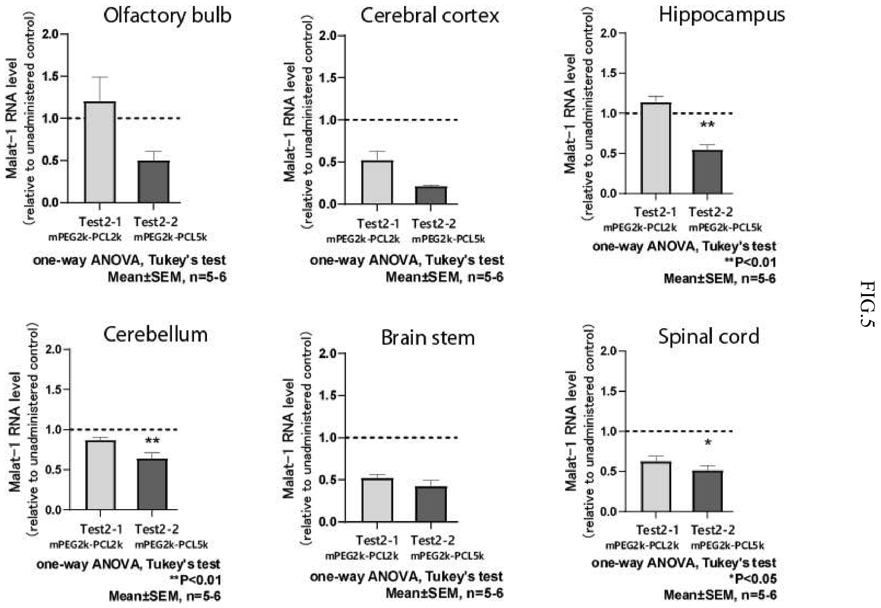

The ΔΔCt value was calculated by subtracting the ΔCt value of the calibrator sample from the ΔCT value of each sample. The relative expression level of the target gene with respect to a unadministered group was calculated using the calculated ΔΔCt value. The results are shown in FIG. 5.

As shown in each graph in FIG. 5, a nucleic acid-containing composition formed from mPEG2k-PCL5k with an average particle diameter of 50 nm or less and a PDI of 0.2 or less (Test 2-2) showed remarkable target gene knock-down activity throughout the brain and spinal cord compared to the nucleic acid-containing composition prepared using mPEG2k-PCL2k (Test 2-1). This revealed that by nasal administration of the nucleic acid-containing composition formed from mPEG2k-PCL5k (Test 2-2), Malat-1-targeting siRNA was delivered to central nervous system tissues and the siRNA silencing action was displayed in a sequence specific manner throughout the central nervous system tissues.

### [Example 4]

### 4. Preparation of antisense oligonucleotide-containing composition (1)/preparation by method including mixing and stirring-ultrafiltration

In this Example, a plurality of nucleic acid-containing compositions containing an antisense oligonucleotide (hereinafter referred to as "ASO") as the nucleic acid were prepared by changing N/P ratios. The block copolymer (I), peptide (II) and nucleic acid forming the nucleic acid-containing compositions are shown in Tables 6 and 7 below. In this Example, an antisense oligonucleotide designed to target Malat-1 gene (manufactured by Gene Design Co., Ltd.) was used as the nucleic acid.

**[Table 6]**

| Test No. | Block copolymer (I) (mole ratio) | Peptide (II) (mole ratio) | Nucleic acid | N/P ratio |
|---|---|---|---|---|
| Test 4 | mPEG5k-PCL10k (0.5 mol) | STR-CH₂R₄H₂C (1 mol) | Malat-1-targeting ASO | 3-30 |

**[Table 7]**

| Malat-1-targeting antisense oligonucleotide | |
|---|---|
| 5'-CTAGTTCACTGAATGC-3' | SEQ ID NO:9 |

| | |
|---|---|
| Underlined nucleic acids are locked nucleic acids (LNAs). All have a phosphorothioate (PS) backbone. | |

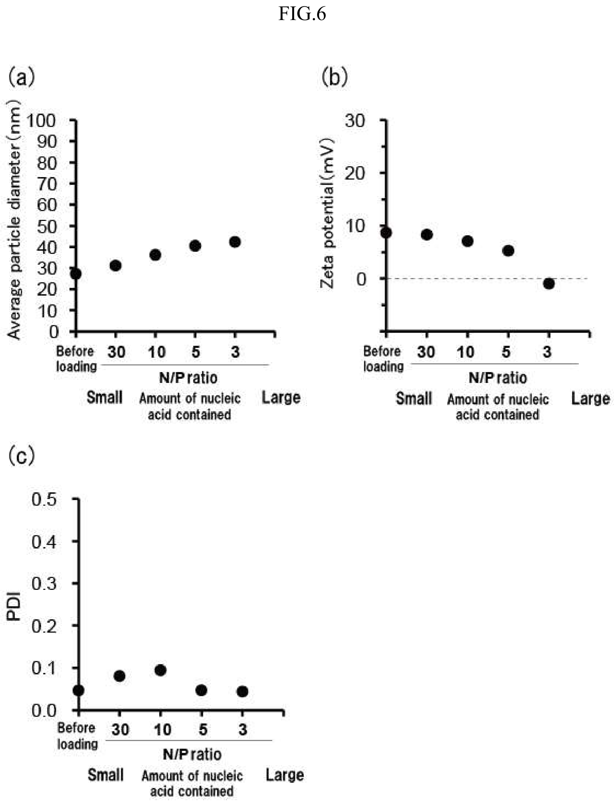

As the nucleic acid (ASO), a 1 mM nucleic acid solution was prepared using nuclease-free water and stored at -80°C. A nucleic acid delivery composition was prepared from the block copolymer (I) and the peptide (II) by the same procedure and method as in Example 1 to obtain a dispersion liquid in which composite particles of the nucleic acid delivery composition were dispersed in the solution. The HEPES buffer solution (10 mM, pH 7.4) was added to the nucleic acid solution so that the amount was equal to the amount of this dispersion liquid. This nucleic acid solution was added dropwise in an equal amount to the dispersion liquid in which composite particles of the nucleic acid delivery composition were dispersed and the obtained mixture was then mildly vortexed and left to stand for 30 minutes to obtain a nucleic acid-containing composition in which composite particles of the nucleic acid, the block copolymer (I) and the peptide (II) were dispersed in the solution. In addition, nucleic acid-containing compositions with an N/P ratio of 3 to 30 were obtained by adjusting the concentration of the nucleic acid solution which was added dropwise to the dispersion liquid in which composite particles of the nucleic acid delivery composition were dispersed. The physical properties such as the average particle diameter (the cumulant average particle diameter by the dynamic light scattering method), polydispersity index (PDI) and zeta potential (particle charge) of composite particles in the obtained nucleic acid-containing compositions were measured by a light scattering analyzer (Zetasizer Ultra manufactured by Malvern Panalytical Ltd.) (n = 4). The measurement temperature was 25°C. In addition, the form of nanoparticles in the dispersion liquid was observed by a transmission electron microscope (TEM). The results are shown in FIG. 6.

The physical properties of the nucleic acid-containing compositions prepared in this Example are shown in the graphs in FIG. 6. The physical properties of composite particles of nucleic acid delivery compositions before loading the nucleic acid had an average particle diameter of 27.3 nm, a PDI of 0.0467 and a zeta potential of 8.66 mV. About physical properties after adding the nucleic acid (ASO), as the amount of nucleic acid contained in the composition increased (the N/P ratio was reduced), the average particle diameter slightly increased and even when the N/P ratio was 3, the average particle diameter was maintained at 50 nm or less. About the zeta potential, meanwhile, as the amount of nucleic acid contained in the composition increased (the N/P ratio was reduced), the zeta potential was reduced and showed negative charge when the N/P ratio was 3. Therefore, it was found that from the viewpoint of the zeta potential, the N/P ratio is preferably above 3, more preferably 4 or more and particularly preferably 5 or more. It should be noted that the value of PDI was small in every N/P ratio, which showed high uniformity.

### [Example 5]

### 5. Study of knock-down effect of antisense oligonucleotide-containing composition by nasal administration (1)

In this Example, the Malat-1-targeting antisense oligonucleotide-containing composition in Test 4 prepared in Example 4 described above was nasally administered to mice and the effect of nucleic acid delivery was examined by verifying whether or not Malat-1 gene constitutively expressed in the brain was knocked down by this ASO using the expression level of Malat-1 gene.

For the administration of the Malat-1-targeting ASO-containing composition, 5-to 6-week-old ICR mice (manufactured by Japan SLC, inc.) were used. The ICR mice were anesthetized by inhalation with isoflurane using a laboratory animal anesthesia device SN-487. The anesthetic concentration was 4% for induction and 2% for maintenance. The mice were put on their backs and the Malat-1-targeting ASO-containing composition prepared as Test 4 was nasally administered using an anesthesia mask for nasal administration (model number: SN-487-70-09 manufactured by SHINANO manufacturing Co., Ltd.) under inhalation anesthesia. The nasal administration was carried out by administering each 2 µL, 20 µL in total, to right and left nasal cavities alternately at an interval of one minute per administration. The dosage of the Malat-1-targeting ASO per administration was 2.36 nmol (12.5 µg)/20 µL. This was nasally administered twice a day (second administration was carried out 6 hours after first administration) every other day for 3 days. The composition concentrations and N/P ratio of the administered Malat-1-targeting ASO-containing composition are each shown below:
- Malat-1-targeting antisense oligonucleotide: 0.118 nmol/µL,
- Concentration of block copolymer (I) (PEG5k-PCL10k): 2.36 nmol/µL,
- Concentration of peptide (II) (STR-CH₂R₄H₂C): 4.72 nmol/µL, and
- N/P ratio: 10.

The mice were euthanized at an anesthetic concentration of 5% a week after completion of first nasal administration and then the brain and spinal cord and peripheral tissues were extracted. The extracted tissues were each washed using 0.9% saline. The brain was divided into the olfactory bulb, hippocampus, mesencephalon and brain stem (combined part of the pons and medulla oblongata). To each of the extracted and divided sites, an RNA extraction reagent (RNAiso manufactured by Takara Bio Inc.) was added, followed by homogenization to extract crude total RNA. To the crude total RNA, DNase (product number: 313-03161 manufactured by NIPPON GENE CO., LTD.) was added and purification was carried out by DNase treatment. To the purified total RNA, a reverse transcriptase (ReverTra Ace (registered trademark) manufactured by TOYOBO Co., Ltd.) was added and a reverse transcription reaction was carried out using a thermal cycler under conditions at 42°C for 40 seconds -> at 99°C for 5 seconds to prepare cDNA.

The obtained cDNA was subjected to qPCR by TaqMan assay. To 2 µL of the prepared cDNA, a qPCR reaction solution (TaqMan Fast Advanced Master Mix manufactured by Thermo Fisher Scientific K.K.), TaqMan Gene Expression Assay (FAM) Malat-1 (Assay ID: Mm01227912_s1 manufactured by Thermo Fisher Scientific K.K.), TaqMan Gene Expression Assay (VIC) β-Actin (Assay ID: Mm02619580_g1 manufactured by Thermo Fisher Scientific K.K.) and nuclease-free water were added and the obtained mixture was mixed by pipetting and subjected to qPCR using a real-time PCR analysis system (CFX connect manufactured by Bio-Rad Laboratories, Inc.) after spin down. The qPCR was carried out by increasing temperature at 95°C for 20 minutes for polymerase activation after incubation at 50°C for two minutes and then repeating [95°C, one sec -> 60°C, 20 sec] for 40 cycles.

The ΔCt value was calculated using the Ct value obtained from the results of qPCR by the following formula:
- ΔCt value = Ct value of target gene (Malat-1) - Ct value of reference gene (β-actin).

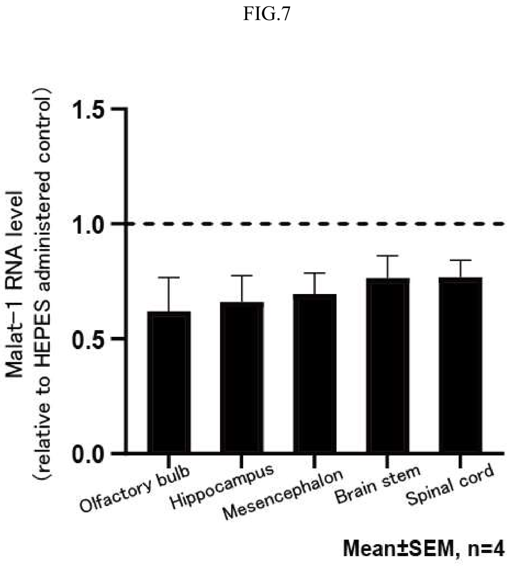

The ΔΔCt value was calculated by subtracting the ΔCt value of the calibrator sample from the ΔCT value of each sample. The relative expression level of the target gene with respect to the unadministered group was calculated using the calculated ΔΔCt value. The results are shown in FIG. 7.

As shown in the graph in FIG. 7, an ASO-containing composition formed from mPEG5k-PCL10k with physical properties of an average particle diameter of 40 nm or less, a PDI of 0.1 or less and a zeta potential of 5 to 10 mV showed a target gene knock-down activity throughout the brain and spinal cord. This revealed that by nasal administration of the ASO-containing composition formed from mPEG5k-PCL10k, Malat-1-targeting ASO was delivered to central nervous system tissues and the silencing action of ASO was displayed in a sequence specific manner throughout central nervous system tissues.

### [Example 6]

### 6. Preparation of antisense oligonucleotide-containing composition (2)/microfluidic device method

In this Example, a plurality of nucleic acid-containing compositions containing an antisense oligonucleotide (ASO) were prepared by changing N/P ratios in a microfluidic device method. The block copolymer (I), peptide (II) and nucleic acid forming the nucleic acid-containing compositions were the same as the combinations used in Examples 4 and 5 (see Tables 6 and 7).

In this Example, ASO-containing compositions were prepared in a microfluidic device method using iLiNP (manufactured by Lilac pharma) which can control the stirring efficiency of each solution in the device. The samples were prepared under conditions of 20°C. The total flow rate (TFR) of a solution flowing in the microfluidic device was set to 250 µL/min. In addition, the flow rate (FRR) of the peptide solution before mixing with respect to the flow rate of the block copolymer solution before mixing was set to 9. The concentration of the block copolymer solution before mixing and the concentration of the peptide solution before mixing were calculated using the following formulae:
- Concentration of block copolymer solution before mixing = final concentration of block copolymer x (FRR + 1), and
- Concentration of peptide solution before mixing = (final concentration of block copolymer x (FRR + 1))/(FRR x mole ratio of block copolymer/peptide).

THF was added to the block copolymer weighed so that the calculated concentration of the block copolymer solution before mixing was obtained and it was completely dissolved using a vortex mixer. To a peptide solution before mixing (obtained by dissolving peptide in a 10 mM, pH 7.4 HEPES buffer solution), 61.7 mol/L DTT with respect to 1 mol/L peptide was added and the obtained mixture was mixed using a vortex mixer to prepare a peptide solution. The block copolymer solution and the peptide solution were each filled in a glass microsyringe. A dispersion liquid of composite particles of the nucleic acid delivery composition was prepared by injection into a nanoparticle manufacturing device iLiNP made of glass using a high accuracy syringe pump (PUMP 11 ELITE manufactured by HARVARD Apparatus) under setting conditions described above. For the obtained dispersion liquid of composite particles of the nucleic acid delivery composition, using a dialysis tube with a molecular weight cut off of 12000 to 14000 (Spectra/Por(registered trademark) 4, 132697 manufactured by Repligen Corporation), dialysis was carried out for 120 minutes by filling a container with the HEPES buffer solution (10 mM, pH 7.4) as the external solution in an amount of 500 times the amount of a sample and then stirring the external solution at 60 rpm with a stirrer.

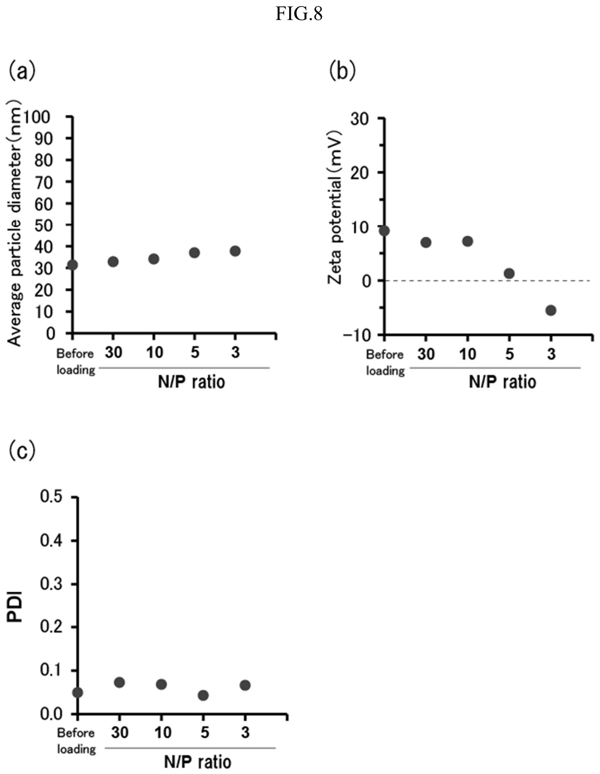

As the nucleic acid (ASO), meanwhile, a 1 mM nucleic acid solution was prepared using nuclease-free water and stored at -80°C. A nucleic acid delivery composition was prepared from the block copolymer (I) and the peptide (II) in the microfluidic device method to obtain a dispersion liquid in which composite particles of the nucleic acid delivery composition was dispersed in the solution. The HEPES buffer solution (10 mM, pH 7.4) was added to the nucleic acid solution so that the amount was equal to the amount of the dispersion liquid. This nucleic acid solution was added dropwise in an equal amount to the dispersion liquid in which composite particles of the nucleic acid delivery composition were dispersed and the obtained mixture was then mildly vortexed and left to stand for 30 minutes to obtain a nucleic acid-containing composition in which composite particles of the nucleic acid, the block copolymer (I) and the peptide (II) were dispersed in the solution. In addition, nucleic acid-containing compositions with an N/P ratio of 3 to 30 were obtained by adjusting the concentration of the nucleic acid solution which was added dropwise to the dispersion liquid in which composite particles of the nucleic acid delivery composition were dispersed. The physical properties such as the average particle diameter (the cumulant average particle diameter by the dynamic light scattering method), polydispersity index (PDI) and zeta potential (particle charge) of composite particles in the obtained nucleic acid-containing compositions were measured by a light scattering analyzer (Zetasizer Ultra manufactured by Malvern Panalytical Ltd.) (n = 4). The measurement temperature was 25°C. In addition, the form of nanoparticles in the dispersion liquid was observed by a transmission electron microscope (TEM). The results are shown in FIG. 8.

The physical properties of the nucleic acid-containing compositions prepared in this Example are shown in the graphs in FIG. 8. The physical properties of composite particles of nucleic acid delivery compositions before loading the nucleic acid had an average particle diameter of 31.3 nm, a PDI of 0.0494 and a zeta potential of 9.11 mV. About physical properties after adding the nucleic acid (ASO), as the amount of nucleic acid contained in the composition increased (the N/P ratio was reduced), the average particle diameter slightly increased and even when the N/P ratio was 3, the average particle diameter was maintained at 40 nm or less. About the zeta potential, meanwhile, as the amount of nucleic acid contained in the composition increased (the N/P ratio was reduced), the zeta potential was reduced and showed negative charge when the N/P ratio was 3. Therefore, it was found that from the viewpoint of the zeta potential, the N/P ratio was preferably 5 or more. It should be noted that the value of PDI was small, 0.1 or less, in every N/P ratio, which showed high uniformity. Furthermore, there were not large differences in the physical properties between the nucleic acid-containing compositions prepared by mixing and stirring and ultrafiltration in Example 4 and the nucleic acid-containing compositions prepared by the microfluidic device method in this Example and thus it was found that the nucleic acid delivery composition and the nucleic acid-containing composition according to the present invention could be also prepared in the microfluidic device method.

### [Example 7]

### 7. Study of knock-down effect of antisense oligonucleotide-containing composition by nasal administration (2)

In this Example, the Malat-1-targeting antisense oligonucleotide-containing compositions prepared by the microfluidic device method in Example 6 described above were nasally administered to mice and the effect of nucleic acid delivery was examined by verifying whether or not Malat-1 gene was knocked down by this ASO using the expression level of Malat-1 gene. The ASO-containing compositions were administered using the same method and procedure as in Example 5. The composition concentrations and N/P ratios of the Malat-1-targeting ASO-containing compositions administered in this Example are each shown below:
- Malat-1-targeting ASO: 0.118 nmol/µL,
- Concentration of block copolymer (I) (PEG5k-PCL10k): 1.18 nmol/µL or 2.36 nmol/µL,
- Concentration of peptide (II) (STR-CH₂R₄H₂C): 2.36 nmol/µL or 4.72 nmol/µL, and
- N/P ratio: 5 or 10.

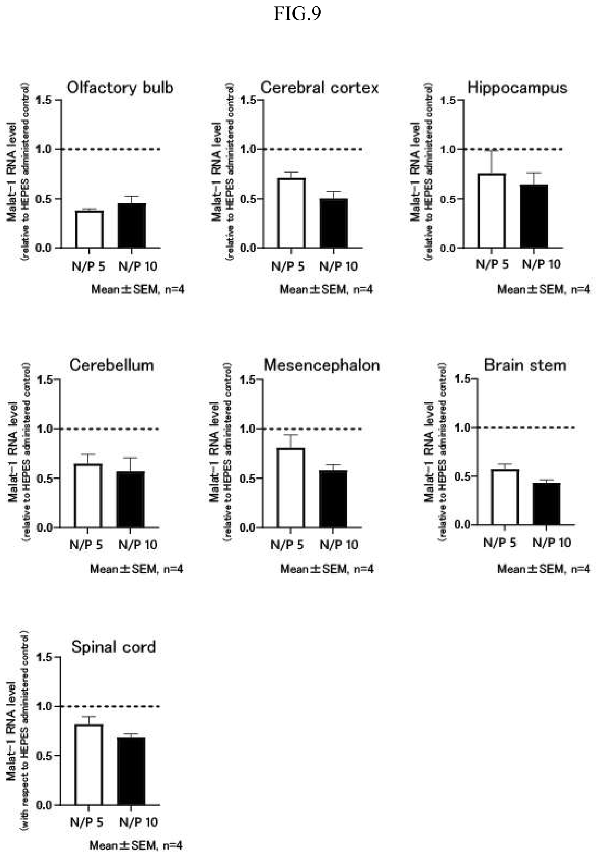

The mice were euthanized at an anesthetic concentration of 5% a week after completion of first nasal administration and then the brain and spinal cord and peripheral tissues were extracted. The extracted tissues were each washed using 0.9% saline. The brain was divided into the olfactory bulb, cerebral cortex, hippocampus, cerebellum, mesencephalon and brain stem (combined part of the pons and medulla oblongata). Total RNA was extracted from each of the extracted and divided sites using the same method as in Example 5 and then cDNA was prepared by the reverse transcription reaction. The obtained cDNA was subjected to qPCR by the TaqMan assay and the expression proportion of the target gene with respect to the unadministered group was calculated. The results are shown in FIG. 9.

As shown in the graphs in FIG. 9, ASO-containing compositions formed from mPEG5k-PCL10k with physical properties of an average particle diameter of 40 nm or less and a PDI of 0.1 or less showed a target gene knock-down activity throughout the brain and spinal cord. Among these, the ASO-containing composition with an N/P ratio = 10 had a higher knock down activity in many central nervous system tissues. This seemed to be because compared to the case of an N/P ratio = 5 the zeta potential at an N/P ratio = 10 was within a range of 5 to 10 mV. This revealed that by nasal administration of the ASO-containing compositions formed from mPEG5k-PCL10k, Malat-1-targeting ASO was delivered to central nervous system tissues and the silencing action of ASO was displayed in a sequence specific manner throughout central nervous system tissues.

### [Example 8]

### 8. Preparation of antisense oligonucleotide-containing composition (3)

In this Example, a plurality of nucleic acid-containing compositions containing a human superoxide dismutase 1 (hSOD1)-targeting antisense oligonucleotide as the nucleic acid were prepared by changing N/P ratios. The block copolymer (I), peptide (II) and nucleic acid forming the nucleic acid-containing compositions are shown in Tables 8 and 9 below. The hSOD1-targeting antisense oligonucleotide used in this Example is an antisense oligonucleotide (manufactured by Cosmo Bio Co., Ltd.) having the same sequence as of QALSODY (Tofersen), a therapeutic agent for amyotrophic lateral sclerosis (ALS).

**[Table 8]**

| Test No. | Block copolymer (I) (mole ratio) | Peptide (II) (mole ratio) | Nucleic acid | N/P ratio |
|---|---|---|---|---|
| Test 8-1 | mPEG2k-PCL5k (1 mol) | STR-CH₂R₄H₂C (1 mol) | hSOD1-targeting ASO | 5-30 |
| Test 8-2 | mPEG5k-PCL10k (0.5 mol) | STR-CH₂R₄H₂C (1 mol) | hSOD1-targeting ASO | 5-30 |

**[Table 9]**

| hSOD1-targeting antisense oligonucleotide | |
|---|---|
| 5'-**CAoGGoA**TACATTTCTA**CoAGoCT**-3' | SEQ ID NO:10 |

| | |
|---|---|
| The **bold** nucleic acids are those modified with 2'-O-methoxyethyl group (MOE). The nucleic acids with "o" are those not modified with phosphorothioate (PS) and the remaining nucleic acids have the PS backbone. | |

As the nucleic acid (ASO), a 1 mM nucleic acid solution was prepared using nuclease-free water and stored at -80°C. A nucleic acid delivery composition was prepared from the block copolymer (I) and the peptide (II) by the same procedure and method as in Example 1 to obtain a dispersion liquid in which composite particles of the nucleic acid delivery composition were dispersed in the solution. The HEPES buffer solution (10 mM, pH 7.4) was added to the nucleic acid solution so that the amount was equal to the amount of this dispersion liquid. This nucleic acid solution was added dropwise in an equal amount to the dispersion liquid in which composite particles of the nucleic acid delivery composition were dispersed and the obtained mixture was then mildly vortexed and left to stand for 30 minutes to obtain a nucleic acid-containing composition in which composite particles of the nucleic acid, the block copolymer (I) and the peptide (II) were dispersed in the solution. In addition, nucleic acid-containing compositions with an N/P ratio of 5 to 30 were obtained by adjusting the concentration of the nucleic acid solution which was added dropwise to the dispersion liquid in which composite particles of the nucleic acid delivery composition were dispersed. The physical properties such as the average particle diameter (the cumulant average particle diameter by the dynamic light scattering method), polydispersity index (PDI) and zeta potential (particle charge) of composite particles in the obtained nucleic acid-containing compositions were measured by a light scattering analyzer (Zetasizer Ultra manufactured by Malvern Panalytical Ltd.) The measurement temperature was 25°C. In addition, the form of nanoparticles in the dispersion liquid was observed by a transmission electron microscope (TEM).

The physical properties of the ASO-containing compositions prepared in this Example are shown in the graphs in FIG. 10(a) and (b). The ASO-containing compositions prepared using mPEG2k-PCL5k as the block copolymer (I) (Test 8-1) and the nucleic acid-containing compositions prepared using mPEG5k-PCL10k (Test 8-2) had an average particle diameter of 50 nm or less and a PDI of 0.3 or less in every N/P ratio and formed particles with high uniformity. About the zeta potential, meanwhile, the ASO-containing compositions prepared using mPEG2k-PCL5k (Test 8-1) had a charge of around 0 when the N/P ratio was 5, while the nucleic acid-containing compositions prepared using mPEG5k-PCL10k (Test 8-2) stably showed positive charge in the range of N/P ratios used in the test. In addition, Fig. 10(c) and Fig. 10(d) are photographs showing nanoparticles in the dispersion liquids of ASO-containing compositions by a transmission electron microscope and both are for observation of the compositions with an N/P ratio of 5. The photographs showed that both mPEG2k-PCL5k (Test 8-1) and mPEG5k-PCL10k (Test 8-2) uniformly formed particles with almost the same size, about 30 to 40 nm.

### [Example 9]

### 9. Study of knock-down effect of antisense oligonucleotide-containing composition by nasal administration (3)

In this Example, the hSOD1-targeting antisense oligonucleotide-containing compositions in Test 8-1 and Test 8-2 prepared in Example 8 described above were nasally administered to amyotrophic lateral sclerosis (ALS) model mice and the effect of nucleic acid delivery was examined by verifying whether or not hSOD1 gene was knocked down by this ASO using the expression level of hSOD1 gene.

For the administration of the hSOD1-targeting ASO-containing compositions, 15-week-old male ALS model mice (human mutated SOD1-G93A transgenic mouse, B6SJL-Tg (SOD1*G93A) 1Gur/J, The Jackson Laboratory) were used. The hSOD1-targeting ASO-containing composition was nasally administered to the ALS model mice using the same method as in Example 5 described above. The nasal administration was carried out by administering each 2 µL, 20 µL in total, to right and left nasal cavities alternately at an interval of one minute per administration. The dosage of the hSOD1-targeting ASO per administration was 10 nmol (71.3 µg)/20 µL. This was nasally administered twice a day (second administration was carried out 6 hours after first administration) every other day for 3 days. The composition concentrations and N/P ratio of the administered hSOD1-targeting ASO-containing compositions are each shown below:
- Concentration of hSOD1-targeting ASO: 0.5 nmol/µL,
- Concentration of block copolymer (I) (PEG2k-PCL5k): 12.5 nmol/µL,
- Concentration of block copolymer (I) (PEG5k-PCL10k): 6.25 nmol/µL,
- Concentration of peptide (II) (STR-CH₂R₄H₂C): 12.5 nmol/µL, and
- N/P ratio: 5.

The mice were euthanized at an anesthetic concentration of 5% a week after completion of first nasal administration and then the brain stem (combined part of the mesencephalon, pons and medulla oblongata) and spinal cord and peripheral tissues were extracted. The extracted tissues were each washed using 0.9% saline. The spinal cord was divided into the upper part of the spinal cord (a region of the cervical spinal cord to thoracic spinal cord) and the lower part of the spinal cord (a region of the lumbar spinal cord to sacral spinal cord). Total RNA was extracted from each of the extracted and divided sites using the same method as in Example 5 and cDNA was then prepared by the reverse transcription reaction.

The obtained cDNA was subjected to qPCR by SYBR (registered trademark) Green assay. To 2 µL of the prepared cDNA, a qPCR reaction solution (SYBR green manufactured by Promega K.K.), the SOD1 primer set of SEQ ID NO:11 and 12 shown in Table 9 below and nuclease-free water were added and the obtained mixture was mixed by pipetting and subjected to qPCR using a real-time PCR analysis system (CFX connect manufactured by Bio-Rad Laboratories, Inc.) after spin down. The qPCR was carried out by increasing temperature at 95°C for 30 seconds for polymerase activation and then repeating [95°C, 30 sec -> 58°C, 10 sec -> 72°C, 20 sec] for 40 cycles. Meanwhile, human glyceraldehyde-3-phosphate dehydrogenase (GAPDH), a housekeeping gene, was selected as an internal reference and qPCR was carried out using the primer set of SEQ ID NO:7 and 8 shown in Table 10 below in place of the SOD1 primer set described above.

**[Table 10]**

| Primer sequence | | |
|---|---|---|
| SOD1 Forward | 5'-CATCAGCCCTAATCCATCTGA-3' | SEQ ID NO:11 |
| SOD1 Reverse | 5'-CGCGACTAACAATCAAAGTGA-3' | SEQ ID NO:12 |
| GAPDH Forward | 5'-TCAACAGCAACTCCCACTCTTCCA-3' | SEQ ID NO:7 |
| GAPDH Reverse | 5'-ACCCTGTTGCTGTAGCCGTATTCA-3' | SEQ ID NO:8 |

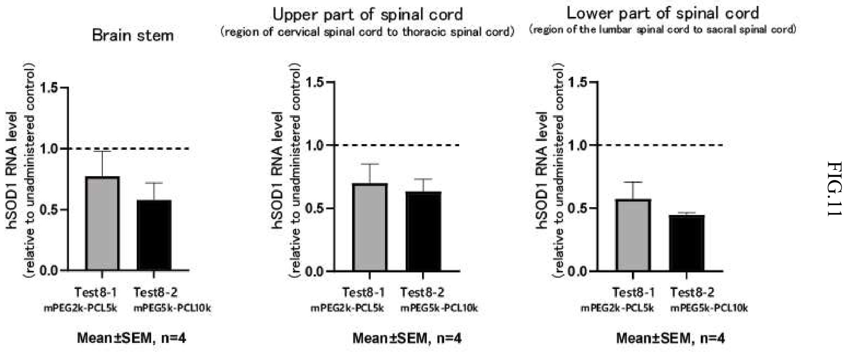

The ΔCt value and ΔΔCt value were calculated using the Ct value obtained from the results of qPCR in the same manner as in Example 5 and the relative expression level of the target gene with respect to the unadministered group was calculated. The results are shown in FIG. 11. As shown in the graphs in FIG. 11, both the ASO-containing composition prepared using mPEG2k-PCL5k as the block copolymer (I) (Test 8-1) and the ASO-containing composition prepared using mPEG5k-PCL10k (Test 8-2) showed a target gene knock down activity throughout the brain and spinal cord. It should be noted that the ASO-containing composition prepared by mPEG5k-PCL10k (Test 8-2) showed a higher knock down activity in the brain stem and the lower part of the spinal cord. This seemed to be because the zeta potential of the ASO-containing composition prepared using mPEG5k-PCL10k (Test 8-2) had positive charge at an N/P ratio of 5 used in the test. This revealed that by nasal administration of the ASO-containing compositions formed from mPEG2k-PCL5k and mPEG5k-PCL10k to the ALS model mice, hSOD1-target ASO was delivered to central nervous system tissues and the silencing action of ASO was displayed in a sequence specific manner throughout central nervous system tissues.

### [Example 10]

### 10. Study of nucleic acid delivery distribution of antisense oligonucleotide-containing composition by nasal administration

In this Example, the hSOD1-targeting antisense oligonucleotide-containing composition prepared by the same materials and procedure as in Example 8 described above and shown in Table 11 below was nasally administered to Macaca fascicularis and the effect of nucleic acid delivery of the compositions according to the present invention by nasal administration was examined by confirming the concentration of the hSOD1-targeting antisense oligonucleotide delivered to central nervous system tissues and the expression level of hSOD1 gene.

**[Table 11]**

| Test No. | Block copolymer (I) (mole ratio) | Peptide (II) (mole ratio) | Nucleic acid | N/P ratio |
|---|---|---|---|---|
| Test 10 | mPEG5k-PCL10k (0.5 mol) | STR-CH₂R₄H₂C (1 mol) | hSOD1-targeting ASO | 3 |

For the administration of the hSOD1-targeting ASO-containing composition, 4 to 7 kg Macaca fascicularis (SHIN NIPPON BIOMEDICAL LABORATORIES, LTD.) were used. For nasal administration, atropine sulfate was intramuscularly administered to Macaca fascicularis for suppressing airway mucus secretion and propofol was intravenously administered thereto for induction of anesthesia. After endotracheal intubation inhalation anesthesia was carried out using a mixed gas of nitrogen gas and oxygen gas (2 : 1) and isoflurane. Macaca fascicularis were put on their backs and an endoscope was inserted into a nasal cavity and kept at the middle turbinate. A catheter was allowed to advance to the cribriform plate of the ethmoid bone and a dispersion liquid of nanoparticles of the hSOD1-targeting ASO-containing composition was dropped, and after completion of administration, Macaca fascicularis were left for 30 minutes. Macaca fascicularis were maintained under anesthesia until being left for 30 minutes is finished. The administration was carried out 3 times for 24 hours (second administration was carried out about 6 hours after first administration and third administration was carried out 24 hours after first administration). The total dosage of the hSOD1-targeting ASO was 19 to 21 mg per animal. The composition concentrations and N/P ratio of the administered hSOD1-targeting ASO-containing composition are each shown below:
- Concentration of hSOD1-targeting ASO: 6.55 µmol (46.7 mg)/mL,
- Concentration of block copolymer (I) (PEG5k-PCL10k): 6.25 µmol/µL,
- Concentration of peptide (II) (STR-CH₂R₄H₂C): 98.25 µmol/mL, and
- N/P ratio: 3.

The brain and spinal cord and the trigeminal nerve were collected as follows. Macaca fascicularis were euthanized under anesthesia 4 hours or 72 hours after completion of third nasal administration and the olfactory bulb and cerebral cortex, striatum, cerebellum, mesencephalon, pons, medulla oblongata, thoracic spinal cord, lumbar spinal cord and trigeminal nerve were then collected and quickly frozen with liquid nitrogen and stored under freezing in an ultra-low temperature freezer at -70°C or lower.

In addition, the cerebrospinal fluid (CSF) was collected as follows. The cerebrospinal fluid (CSF) was collected by puncture into the lumbar vertebrae under combined anesthesia by intramuscular administration of ketamine hydrochloride or ketamine and medetomidine hydrochloride 8 hours, 24 hours or 72 hours after completion of third nasal administration. The collected amount was about 0.2 mL. The collected CSF was quickly cooled on ice and then centrifuged (4°C, 1700 xg, 5 minutes) to remove blood cell components. About 0.1 mL of the supernatant was dispensed to sample tubes and stored under freezing in an ultra-low temperature freezer at -70°C or lower.

### [Measurement of concentration of hSOD1-targeting antisense oligonucleotide delivered to central nervous system tissue]

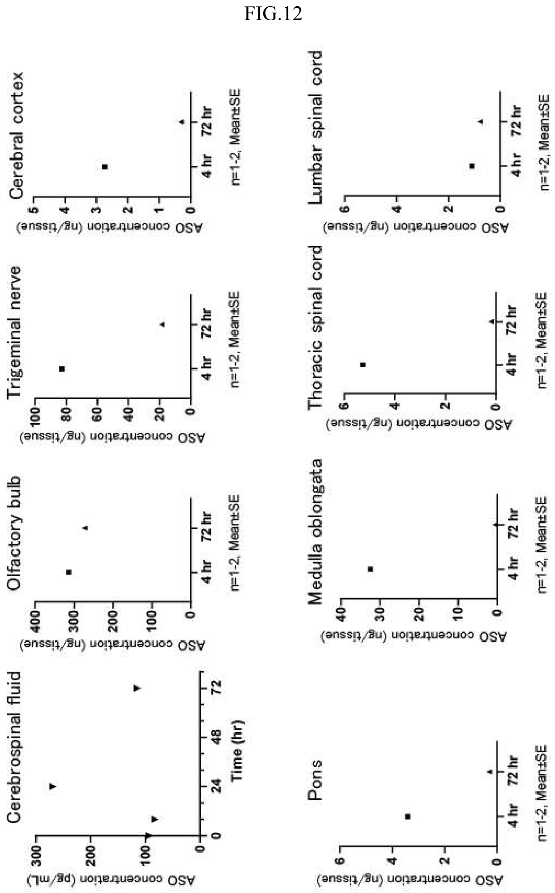

A nucleic acid extraction reagent (RNAiso manufactured by Takara Bio Inc.) was added to each of the collected tissues, followed by homogenization. The supernatant was then collected by centrifugation to obtain tissue lysate. The amount of hSOD1-targeting ASO contained in each sample was measured by a hybridization ELISA method using the tissue lysate sample and cerebrospinal fluid sample. To a PCR plate, 50 µL each of a template probe (5 nM) having a complementary sequence to the hSOD1-targeting ASO was added and mixed with each of the sample solutions or the reference solution. The PCR plate was sealed and hybridized by a thermal cycler under conditions of 95°C, 5 min -> 42°C, 1.5 h. To the assay plate after blocking treatment using a blocking buffer (SuperBlock Blocking Buffer manufactured by Thermo Fisher Scientific K.K.), 40 µL each of the sample solutions and the reference solution after hybridization was added and the plate was incubated at 37°C for 60 minutes. Meanwhile, a detection probe (100 µM, 4.08 µL), T4 Ligase (400 U/µL, 0.6 µL), a 10X reaction buffer (300 µL) and ddH₂O (5694 µL) were mixed to prepare a detection probe solution. The assay plate was washed with a wash buffer 6 times, 50 µL each of the detection probe solution was then added thereto and the plate was incubated at 25°C for 60 minutes for ligation and then stored at 4°C overnight. Meanwhile, an S1 nuclease (75 U/µL, 1.005 µL), a 10X nuclease buffer (75 µL) and ddH₂O (675 µL) were mixed to obtain a solution A. A 10X nuclease buffer (608 µL) and ddH₂O (5467 µL) were mixed and 675 µL of the solution A was added thereto to prepare an S1 nuclease solution. The assay plate after ligation was washed 6 times and 50 µL each of the S1 nuclease solution was then added thereto. The plate was sealed and incubated at 37°C for 30 minutes. Next, anti-digoxigenin-AP, Fab fragments (3 µL), a blocking buffer (600 µL) and ddH₂O (5397 µL) were mixed to prepare a labelling antibody solution. The plate after nuclease treatment was washed 6 times, 50 µL each of the labeling antibody solution was added thereto and the plate was incubated at 25°C for 30 minutes. Next, the plate after incubation was washed 6 times, a substrate solution (QuantaBlu Fluorogenic Peroxidase Substrate Kits manufactured by Thermo Fisher Scientific K.K.) was added thereto and the plate was incubated at 25°C for 15 minutes. After the reaction, 90 µL each of the solution in each well of the assay plate was transferred to a measurement plate and the fluorescence intensity was measured at an excitation wavelength of 325 nm and a detection wavelength of 420 nm using a microplate reader (SpectraMax iD3 manufactured by Molecular Devices). The concentration of hSOD1-targeting ASO in each sample was calculated from the information of the calibration curve based on the reference solution. The results are shown in FIG. 12.

In the nose-to-brain route by nasal administration a drug is directly delivered from nasal mucosa to central nervous systems via the olfactory nerve and trigeminal nerve. According to each graph in FIG. 12, as this is supported, it was revealed that the concentration of hSOD1-targeting ASO was very high in the olfactory bulb and trigeminal nerve 4 hours after nasal administration and hSOD1-targeting ASO was distributed in the concentration in nanograms per tissue in other tissues. On the other hand, the concentration of the hSOD1-targeting ASO was highest 24 hours after the nasal administration in the cerebrospinal fluid and this is considered to mean that ASO delivered to each central nervous system tissue was discharged via the cerebrospinal fluid. This revealed that by nasal administration of the ASO-containing composition formed from the nucleic acid delivery composition according to the present invention, hSOD1-targeting ASO was delivered to central nervous system tissues with high efficiency and distributed at a high concentration throughout central nervous system tissues and then discharged via the cerebrospinal fluid.

### [Measurement of expression level of hSOD1 gene]

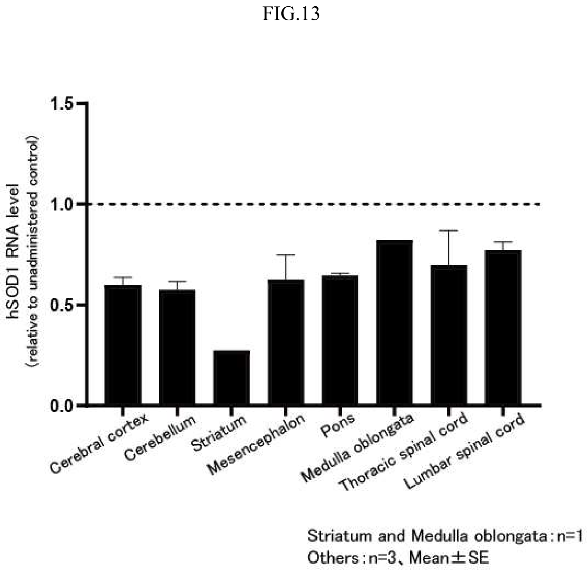

Total RNA was extracted from each of the extracted and divided sites using the same method as in Example 5 and cDNA was then prepared by the reverse transcription reaction. The obtained cDNA was subjected to qPCR by TaqMan assay and the expression proportion of the target gene with respect to the unadministered group was calculated. It should be noted that in the qPCR reaction solution used for the TaqMan assay, TaqMan Gene Expression Assay (FAM) SOD1 (Assay ID: Mf04363557_m1 manufactured by Thermo Fisher Scientific K.K.) and TaqMan Gene Expression Assay (VIC) β-Actin (Assay ID: Mf04354341_g1 manufactured by Thermo Fisher Scientific K.K.) were used as the primer and probe. The results are shown in FIG. 13.

As shown in the graph in FIG. 13, the hSOD1-targeting ASO-containing composition prepared in this Example showed a target gene knock-down activity throughout the brain and spinal cord. This revealed that by nasal administration of the ASO-containing composition formed from the nucleic acid delivery composition according to the present invention, hSOD1-targeting ASO was delivered to central nervous system tissues and the silencing action of ASO was displayed in a sequence specific manner throughout central nervous system tissues.

The present invention is not limited to the embodiments or Examples described above, and also encompasses various modified designs in the technical scope without departing from the spirit of the invention described in claims.

### INDUSTRIAL APPLICABILITY

The present invention provides a nucleic acid delivery composition and a nucleic acid-containing composition which can deliver a nucleic acid to central nervous system tissues such as the brain and spinal cord by nasal administration, and the nucleic acid delivery composition and the nucleic acid-containing composition of the present invention can be used as a medicine for treating central nervous system tissues as an example and thus are widely useful in industries such as the medicine and medical fields.

## Claims

1. A nucleic acid delivery composition used to deliver a nucleic acid to central nervous system tissues or trigeminal nerve, comprising
a block copolymer (I) having a polyethylene glycol segment (a) and a polycaprolactone segment (b), and
a peptide (II) comprising 8 to 20 amino acid residues modified with a fatty acid,
wherein in the block copolymer (I)
an average molecular weight Ma of the polyethylene glycol segment (a) is 1,500 to 2,500 or 3,500 to 6,500,
an average molecular weight Mb of the polycaprolactone segment (b) is 1.5 to 3 times the average molecular weight Ma of the polyethylene glycol segment (a), and
the nucleic acid delivery composition is delivered to the central nervous system tissues or trigeminal nerve by nasal administration.

2. The nucleic acid delivery composition according to claim 1, wherein in the block copolymer (I), the average molecular weight Mb of the polycaprolactone segment (b) is 3,500 to 6,500 or 8,000 to 12,000.

3. The nucleic acid delivery composition according to claim 1, wherein a mixing ratio of the block copolymer (I) and the peptide (II) is (I) : (II) = 0.25 : 1 to 2 : 1 by mole ratio.

4. The nucleic acid delivery composition according to claim 1, wherein the block copolymer (I) is methoxypolyethylene glycol-poly(ε-caprolactone).

5. The nucleic acid delivery composition according to claim 1, wherein the peptide (II) is a peptide comprising cysteine residues, histidine residues and arginine residues, and the fatty acid modifying the peptide is stearic acid.

6. The nucleic acid delivery composition according to claim 1, wherein the block copolymer (I) and the peptide (II) form nanoparticles.

7. The nucleic acid delivery composition according to claim 6, wherein the nanoparticles have an average particle diameter of 50 nm or less, a polydispersity index (PDI) of 0.3 or less and a zeta potential of 1 to 20 mV.

8. A nucleic acid-containing composition, further comprising a nucleic acid in the nucleic acid delivery composition according to any one of claims 1 to 7.

9. The nucleic acid-containing composition according to claim 8, wherein a ratio (N/P ratio) of a total number of moles (N) of cationic groups derived from the peptide (II) and a total number of moles (P) of phosphate groups derived from the nucleic acid is 3 to 30.

10. The nucleic acid-containing composition according to claim 8, wherein the nucleic acid is an antisense oligonucleotide, siRNA or mRNA for treating a disease in central nervous system tissues.

11. A medicine comprising the nucleic acid-containing composition according to claim 8 as an active ingredient.
